# EUROPEAN PATENT APPLICATION

(11) **EP 4 725 953 A1**
(43) Date of publication of application: **15.04.2026**
(21) Application number: 24206543.1
(22) Date of filing: 14.10.2024
(51) Int. Cl.: C07K 7/06, A61K 38/00

(54) **SELF-ASSEMBLING PEPTIDES AND HYDROGELS**

(71) Applicant: Max-Planck-Gesellschaft zur Förderung der Wissenschaften e.V., 80539 München (DE); Universität Ulm, 89081 Ulm (DE)
(72) Inventor: The designation of the inventor has not yet been filed
(74) Representative: Arth, Hans-Lothar

(57) **Abstract**

The present invention relates to self-assembling peptides consisting of at least 6 amino acids and containing the amino acid sequence **X¹-A⁴-A⁵-A⁶-X²**. Said peptides self-assemble into fibrils especially under physiological conditions in an aqueous dispersion phase thereby forming a hydrogel and are thus particularly useful for forming a hydrogel composition.

## Description

### Field of the invention

The present invention relates to self-assembling peptides consisting of at least 6 amino acids and containing the amino acid sequence X¹-A⁴-A⁵-A⁶-X². Said peptides self-assemble into fibrils especially under physiological conditions in an aqueous dispersion phase thereby forming a hydrogel and are thus particularly useful for forming a hydrogel composition.

### Background of the invention

Hydrogels are three-dimensional polymeric networks capable of retaining large amounts of water, making them highly versatile materials for various biomedical and industrial applications. Due to their special properties, hydrogels are used in many areas, including drug delivery systems,tissue engineering, wound healing, contact lenses, biosensors, agriculture, 3D bioprinting, hygiene products, and food supplements.

Among the different classes of hydrogels, peptide-based hydrogels have garnered significant attention due to their biocompatibility, tunable properties, and ability to self-assemble under physiological conditions. Peptides, as building blocks, offer unique advantages, such as molecular recognition, biodegradability, and the ability to form specific secondary structures, which can be leveraged to create hydrogels with tailored mechanical and chemical characteristics. However, the production of peptide hydrogels can be costly and complex, particularly for large-scale applications, due to the need for precise peptide synthesis and purification techniques.

Therefore, it is the objective of the present invention to provide a novel class of peptide hydrogels with versatile mechanical properties that can be tailored to various uses in diverse fields.

The objective of the present invention is solved by the teaching of the independent claims. Further advantageous features, aspects and details of the invention are evident from the dependent claims, the description, the figures, and the examples of the present application.

### Brief description of the invention

The present invention is directed to a self-assembling peptide comprising or consisting of at least 6 amino acids having the following amino acid sequence of general formula (I):

X¹-A⁴-A⁵-A⁶-X² (I)

wherein
X¹ represents -H, A³-, A²-A³-, or A¹-A²-A³-;
X² represents -H, -A⁷, -A⁷-A⁸, -A⁷-A⁸-A⁹, -A⁷-A⁸-A⁹-A¹⁰, or -A⁷-A⁸-A⁹-A¹⁰-A¹¹;
A¹ and A⁴ represent independently of each other a small neutral amino acid or a neutral hydrophilic amino acid;
A² and A⁵ represent independently of each other a neutral amino acid, an aromatic amino acid or an amino acid amide;
A³ represents a basic amino acid, a large neutral amino acid, or an amino acid amide;
A⁶ represents an amino acid selected from S and T;
A⁷ and A⁸ represent independently of each other a neutral amino acid;
A⁹ represents independently of each other a basic amino acid, a large neutral amino acid, or an amino acid amide;
A¹⁰ represents a basic amino acid, or an aromatic amino acid;
A¹¹ represents a neutral amino acid, a hydrophilic amino acid, or an amino acid amid.

The inventors have surprisingly found that the inventive short peptides of the sequence X¹-A⁴-A⁵-A⁶-X² self-assemble into fibrils, especially self-assemble into fibrils under physiological conditions. Using rheology experiments and TEM imaging, the inventors examined the influence of sequence variations, pH, and peptide concentration on the mechanical properties of the herein disclosed hydrogels. The experimental data demonstrate that the inventive peptides form robust hydrogels (e.g. from about 10 to about 100,000 Pa storage modulus) at low weight percent.

In a preferred embodiment, the self-assembling peptide consists of at least 6 amino acids having the following amino acid sequence of general formula (I):

X¹-A⁴-A⁵-A⁶-X² (I)

wherein
X¹ represents -H, A³-, A²-A³-, or A¹-A²-A³-;
X² represents -H, -A⁷, -A⁷-A⁸, -A⁷-A⁸-A⁹, -A⁷-A⁸-A⁹-A¹⁰, or -A⁷-A⁸-A⁹-A¹⁰-A¹¹;
A¹ represents an amino acid selected from G and A;
A² represents an amino acid selected from Y, F, and W;
A³ represents an amino acid selected from R, K and L;
A⁴ represents an amino acid selected from G, and A;
A⁵ represents an amino acid selected from I, Y, Q, N, L, and F;
A⁶ represents an amino acid selected from S and T;
A⁷ represents an amino acid selected from L, I, and V;
A⁸ represents an amino acid selected from A, G, and V;
A⁹ represents an amino acid selected from K, N, and Q;
A¹⁰ represents an amino acid selected from W, and F;
A¹¹ represents an amino acid selected from M, M-NH₂, L, I, V, A, and G.

In a preferred embodiment, the self-assembling peptide consists of at least 6 amino acids having the following amino acid sequence of general formula (I):

X¹-A⁴-A⁵-A⁶-X² (I)

wherein
X¹ represents -H, A³-, A²-A³-, or A¹-A²-A³-;
X² represents -H, -A⁷, -A⁷-A⁸, -A⁷-A⁸-A⁹, -A⁷-A⁸-A⁹-A¹⁰, or -A⁷-A⁸-A⁹-A¹⁰-A¹¹;
A¹ represents G;
A² represents an amino acid selected from Y, and F;
A³ represents an amino acid selected from R, K and L;
A⁴ represents G;
A⁵ represents an amino acid selected from I, and Y;
A⁶ represents an amino acid selected from S and T;
A⁷ represents L;
A⁸ represents A;
A⁹ represents an amino acid selected from K and N;
A¹⁰ represents W;
A¹¹ represents an amino acid selected from M, M-NH₂, and V.

In a further preferred embodiment, the self-assembling peptide according to the invention consists of at least 6 amino acids having the following amino acid sequence of general formula (I):

X¹-A⁴-A⁵-A⁶-X² (I)

wherein
X¹ represents -H, A³-, A²-A³- or A¹-A²-A³-;
X² represents -H, -A⁷, -A⁷-A⁸, -A⁷-A⁸-A⁹, -A⁷-A⁸-A⁹-A¹⁰, or -A⁷-A⁸-A⁹-A¹⁰-A¹¹;
and A¹ to A¹¹ have the meanings as defined herein.

In a further preferred embodiment, the self-assembling peptide according to the invention consists of the amino acid sequence selected from: GYLGIS (SEQ ID NO: 2), GISLAN (SEQ ID NO: 3), YRGISLANWM (SEQ ID NO: 4), YRGISLANW (SEQ ID NO: 5), GFRGISLANWM (SEQ ID NO: 6), GYKGISLANWM (SEQ ID NO: 7), GYRGISLANWM (SEQ ID NO: 8), GYRGYSLANWM (SEQ ID NO: 9), GYRGITLANWΛΛ (SEQ ID NO: 10), GYRGISLAKWM (SEQ ID NO: 11) GYRGISLANWV (SEQ ID NO: 12), GYLGISLANW (SEQ ID NO: 13), and GYRGISLANWM-NH₂ (SEQ ID NO: 14).

In a further preferred embodiment, the self-assembling peptide according to the invention has the amino acid sequence GYRGISLANWM (SEQ ID NO: 8).

A further aspect of the present invention is directed to a hydrogel composition comprising an aqueous dispersion phase comprising an aqueous dispersion medium; and at least one self-assembling peptide as disclosed herein, wherein the hydrogel is formed by non-covalent interactions of said self-assembling peptide in said aqueous dispersion phase.

In a preferred embodiment, the hydrogel composition comprises an aqueous dispersion phase comprising an aqueous dispersion medium; and at least one self-assembling peptide as disclosed herein, wherein the hydrogel is formed by non-covalent interactions and most probably by self-assembly of said self-assembling peptide in said aqueous dispersion phase, and wherein the self-assembling peptide is present in said hydrogel at a level of from about 0.01% by weight to about 20%, preferably from about 0.01% by weight to about 10% by weight, based upon the total weight of the hydrogel.

In a preferred embodiment, the hydrogel composition comprises an aqueous dispersion phase comprising preferably a physiologically acceptable aqueous dispersion medium; and at least one self-assembling peptide as disclosed herein, wherein the hydrogel is formed by non-covalent interactions, probably by self-assembly of said self-assembling peptide in said aqueous dispersion phase.

In a preferred embodiment, the hydrogel composition comprises an aqueous dispersion phase comprising an aqueous dispersion medium; and at least one self-assembling peptide as disclosed herein, wherein the hydrogel is formed by non-covalent interactions of said self-assembling peptide in said aqueous dispersion phase, wherein the aqueous dispersion medium comprises one or more salts selected from (NH₄)₂SO₄, Na₂SO₄, NaCl, KCl, CH₃COONH₄, NH₃C(CH₂OH)₃Cl, KH₂PO₄, and Na₂HPO₄.

The following buffers are preferred: acetate buffer, ammonium buffer (NH₃ / NH₄⁺), Tris-HCl buffer, NH₂C(CH₂OH)₃ / NH₃⁺C(CH₂OH)₃ buffer, TAE buffer, phosphate buffer, and phosphate buffered saline.

In a preferred embodiment, the hydrogel composition comprises an aqueous dispersion phase comprising an aqueous dispersion medium; and at least one self-assembling peptide as disclosed herein, wherein the hydrogel is formed by non-covalent interactions of said self-assembling peptide in said aqueous dispersion phase, wherein the composition has a pH value between 4.0 and 9.0, preferably between 4.5 and 8.5, more preferably between 4.8 and 8.0, still more preferably between 5.0 and 7.5.

In a preferred embodiment, the hydrogel composition comprises an aqueous dispersion phase comprising an aqueous dispersion medium; and at least one self-assembling peptide as disclosed herein, wherein the hydrogel is formed by non-covalent interactions of said self-assembling peptide in said aqueous dispersion phase, wherein the hydrogel has a pH value between 4.0 and 8.5, preferably between 4.5 and 8.0.

In a preferred embodiment, the hydrogel composition comprises an aqueous dispersion phase comprising an aqueous dispersion medium; and at least one self-assembling peptide as disclosed herein, wherein the hydrogel is formed by non-covalent interactions of said self-assembling peptide in said aqueous dispersion phase, and wherein the composition has an ionic strength between 0.0001 M and 1.5 M, preferably between 0.001 M and 0.5 M, more preferably 0.005 M and 0.3 M.

The inventive self-assembling covers a broad range of storage moduli from 10 Pa to 100,000 Pa. Thus, in a preferred embodiment, the hydrogel composition comprises an aqueous dispersion phase comprising an aqueous dispersion medium; and at least one self-assembling peptide as disclosed herein, wherein the hydrogel is formed by non-covalent interactions of said self-assembling peptide in said aqueous dispersion phase, and wherein the composition has a storage modulus in the range of about 10 Pa to about 100,000 Pa.

### Description of the invention

### Definitions

Throughout this specification amino acid residues will be denoted by the three-letter abbreviation or single-letter code as follows:

| **Amino Acid** | **Three-letter abbreviation** | **One-letter Symbol** |
|---|---|---|
| Alanine | Ala | A |
| Arginine | Arg | R |
| Asparagine | Asn | N |
| Aspartic Acid | Asp | D |
| Cysteine | Cys | C |
| Glutamine | Gln | Q |
| Glutamic Acid | Glu | E |
| Glycine | Gly | G |
| Histidine | His | H |
| Isoleucine | Ile | I |
| Leucine | Leu | L |
| Lysine | Lys | K |
| Methionine | Met | M |
| Phenylalanine | Phe | F |
| Proline | Pro | P |
| Serine | Ser | S |
| Threonine | Thr | T |
| Tryptophan | Trp | W |
| Tyrosine | Tyr | Y |
| Valine | Val | V |

The term **"neutral amino acid",** as used herein, refers to any proteinogenic and non-proteinogenic amino acid having an aliphatic or cyclic side chain or no side chain, including but not limiting to glycine (Gly, G), alanine (Ala, A), proline (Pro, P), leucine (Leu, L), valine (Val, V), isoleucine (Ile, I), and methionine (Met, M).

The term **"small neutral amino acid",** as used herein, refers to any proteinogenic and non-proteinogenic neutral amino acid having a volume of less then 120 Å³, including but not limiting to glycine (Gly, G), alanine (Ala, A), and proline (Pro, P).

The term **"large neutral amino acid",** as used herein, refers to any proteinogenic and non-proteinogenic neutral amino acid having a volume of more then 120 Å³ (van der Waals volume Å³; 1 Å = 100 pm = 0.1 nm = 10⁻¹⁰ m), including but not limiting to leucine (Leu, L), valine (Val, V), isoleucine (Ile, I), and methionine (Met, M).

The term **"neutral hydrophilic amino acid",** as used herein, refers to any proteinogenic and non-proteinogenic hydrophilic amino acid having a protic functional group, but lacking an acidic or basic group, including but not limiting to serine (Ser, S), threonine (Thr, T), and cysteine (Cys, C).

The term **"aromatic amino acid",** as used herein, refers to any proteinogenic and non-proteinogenic amino acid having an aromatic group, including but not limiting to phenylalanine (Phe, F), tryptophane (Trp, W), and tyrosine (Tyr, Y).

The term **"amino acid amide",** as used herein, refers to any proteinogenic and non-proteinogenic hydrophilic amino acid having an amide functional group in the side chain, including but not limiting to asparagine (Asn, N), and glutamine (Gln, Q).

The term **"acidic amino acid"** refers to any proteinogenic and non-proteinogenic amino acid having an acidic (carboxy) group in the side chain, including but not limiting to aspartic acid (Asp, D), and glutamic acid (Glu, E).

The term **"basic amino acid",** as used herein, refers to any proteinogenic and non-proteinogenic amino acid having a basic (amino) group in the side chain, including but not limiting to lysine (Lys, K), arginine (Arg, R), and histidine(His, H).

Surprisingly it was found that a self-assembling peptide consisting of at least 6 amino acids having the following amino acid sequence of general formula (I):

**X¹-A⁴-A⁵-A⁶-X²** **(I)**

wherein
**X¹** represents -H, **A³-, A²-A³- or A¹-A²-A³-;**
**X²** represents -H, **-A⁷, -A⁷-A⁸, -A⁷-A⁸-A⁹, -A⁷-A⁸-A⁹-A¹⁰,** or **-A⁷-A⁸-A⁹-A¹⁰-A¹¹;**
**A¹** and **A⁴** represent independently of each other a small neutral amino acid or a neutral hydrophilic amino acid;
**A²** and **A⁵** represent independently of each other a neutral amino acid, an aromatic amino acid or an amino acid amide;
**A³** represents a basic amino acid, a large neutral amino acid, or an amino acid amide;
**A⁶** represents an amino acid selected from S and T;
**A⁷** and **A⁸** represent independently of each other a neutral amino acid;
**A⁹** represents a basic amino acid, or an amino acid amide;
**A¹⁰** represents a basic amino acid, or an aromatic amino acid;
**A¹¹** represents a neutral amino acid, a hydrophilic amino acid, or an amino acid amide, solves the objective of the present invention in an optimal way.

The self-assembling peptides according to the present invention consist of at least 6 amino acids. For example, excluded from the scope of the present application are peptides of the general formulae **H-A⁴-A⁵-A⁶-H, H-A³-A⁴-A⁵-A⁶-H, H-A⁴-A⁵-A⁶**-**A⁷-H, H-A²-A³-A⁴-A⁵-A⁶-H, H-A⁴-A⁵-A⁶-A⁷-A⁸-H** and **H-A³-A⁴-A⁵-A⁶-A⁷-H.**

Moreover, formulated differently, the self-assembling peptides according to the present invention consist of 6 to 11 amino acids. In some preferred embodiments, the self-assembling peptides according to the present invention consist of at least 7 amino acids, more preferably at least 8 amino acids, more preferably at least 9 amino acids, even more preferably at least 10 amino acids. In particular preferred embodiments, the self-assembling peptides according to the present invention consist of 11 amino acids. Furthermore, in some embodiments, wherein the self-assembling peptides according to the present invention consist of 9 - 11 amino acids, it is also preferred that A³ represents a basic amino acid, or an amino acid amide, wherein the amide is in the side chain.

Preferred are self-assembling peptides consisting of at least 6 amino acids having the amino acid sequence of general formula (I), wherein
**A¹** represents an amino acid selected from G and A;
**A²** represents an amino acid selected from Y, F, and W;
**A³** represents an amino acid selected from R, K and L;
**A⁴** represents an amino acid selected from G, and A;
**A⁵** represents an amino acid selected from I, Y, Q, N, L, and F;
**A⁶** represents an amino acid selected from S and T;
**A⁷** represents an amino acid selected from L, I, and V;
**A⁸** represents an amino acid selected from A, G, and V;
**A⁹** represents an amino acid selected from K, N, and Q;
**A¹⁰** represents an amino acid selected from W, and F;
**A¹¹** represents an amino acid selected from M, M-NH₂, L, I, V, A, and G;
and more preferably
**A'** represents an amino acid selected from G and A;
**A²** represents an amino acid selected from Y, and F;
**A³** represents an amino acid selected from R, K and L;
**A⁴** represents an amino acid selected from G, and A;
**A⁵** represents an amino acid selected from I, and Y;
**A⁶** represents an amino acid selected from S and T;
**A⁷** represents an amino acid selected from L, and I;
**A⁸** represents an amino acid selected from A, and G;
**A⁹** represents an amino acid selected from K and N;
**A¹⁰** represents an amino acid selected from W, and F;
**A¹¹** represents an amino acid selected from M, M-NH₂, L, I, and V;
and still more preferably
**A¹** represents G;
**A²** represents an amino acid selected from Y, and F;
**A³** represents an amino acid selected from R, K and L;
**A⁴** represents G;
**A⁵** represents an amino acid selected from I, and Y;
**A⁶** represents an amino acid selected from S and T;
**A⁷** represents L;
**A⁸** represents A;
**A⁹** represents an amino acid selected from K and N;
**A¹⁰** represents W;
**A¹¹** represents an amino acid selected from M, M-NH₂, and V.

Preferred are self-assembling peptides consisting of at least 6 amino acids having the general formula (I) **X¹-A⁴-A⁵-A⁶-X²,**
wherein **X¹** represents -H and **X²** represents **-A⁷-A⁸-A⁹, -A⁷-A⁸-A⁹-A¹⁰,** or **-A⁷-A⁸-A⁹-A¹⁰-A¹¹;**
or wherein
**X²** represents -H and **X¹** represents **A¹-A²-A³-;**
and the residues **A¹ - A¹¹** have the meanings as disclosed herein.

Preferred are self-assembling peptides consisting of at least 6 amino acids having the general formula (I) **X¹-A⁴-A⁵-A⁶-X²,**
wherein **X¹** represents A³- and **X²** represents **-A⁷-A⁸, -A⁷-A⁸-A⁹, -A⁷-A⁸-A⁹-A¹⁰,** or **-A⁷-A⁸-A⁹-A¹⁰-A¹¹;**
or wherein
**X²** represents **-A⁷,** and **X¹** represents **A¹-A²-A³-,** or **A²-A³-;**
and the residues **A¹ - A¹¹** have the meanings as disclosed herein.

Preferred are self-assembling peptides consisting of at least 6 amino acids having the general formula (I) **X¹-A⁴-A⁵-A⁶-X²,**
wherein **X¹** represents **A²-A³-** and **X²** represents **-A⁷, -A⁷-A⁸, -A⁷-A⁸-A⁹, -A⁷-A⁸-A⁹-A¹⁰,** or **-A⁷-A⁸-A⁹-A¹⁰-A¹¹;**
or wherein
**X²** represents **-A⁷-A⁸** and **X¹** represents **A¹-A²-A³-, A²-A³- or A³-;**
and the residues **A¹ - A¹¹** have the meanings as disclosed herein.

In some embodiments, the present invention relates to a self-assembling peptide consisting of at least 6 amino acids having the following amino acid sequence (SEQ ID NO: 1):

**X^{1'}-G-A⁵-S-L-A-N-X^{7'}**

wherein
**X^{1'}** represents -H or G-Y-A³- ;
**A⁵** represents the amino acid I or Y;
**X^{7'}** represents a -H or **-W-X⁷";**
**A³** represents the amino acid K, R, or L;
**X⁷"** represents -H or **-A¹¹;** and
**A¹¹** represents the amino acid M, M-NH₂, or V.

Furthermore, self-assembling peptides consisting of at least 6 amino acids having the following general formulae are preferred:

**X¹-A⁴-A⁵-A⁶-A⁷-X³ ,**

**X¹-A⁴-A⁵-A⁶-A⁷-A⁸-X⁵** ,

**X¹-A⁴-A⁵-A⁶-A⁷-A⁸-A⁹-X⁷ ,**

**X¹-A⁴-A⁵-A⁶-A⁷-A⁸-A⁹-A¹⁰-X⁸,**

**X¹-A⁴-A⁵-A⁶-A⁷-A⁸-A⁹-A¹⁰-A¹¹ ,**

**X⁴-A³-A⁴-A⁵-A⁶-X² ,**

**X⁶-A²-A³-A⁴-A⁵-A⁶-X² ,**

**A¹-A²-A³-A⁴-A⁵-A⁶-X² ,**

**X⁴-A³-A⁴-A⁵-A⁶-A⁷-X³ ,**

**X⁶-A²-A³-A⁴-A⁵-A⁶-A⁷-X³ ,**

**X⁴-A³-A⁴-A⁵-A⁶-A⁷-A⁸-X⁵ ,**

wherein
**X¹** represents -H, **A¹-A²-A³- , A²-A³-,** or **A³-;**
**X²** represents -H, **-A⁷, -A⁷-A⁸, -A⁷-A⁸-A⁹, -A⁷-A⁸-A⁹-A¹⁰,** or **-A⁷-A⁸-A⁹-A¹⁰-A**^{**1**1} ;
**X³** represents -H, **-A⁸, -A⁸-A⁹, -A⁸-A⁹-A¹⁰,** or **-A⁸-A⁹-A¹⁰-A¹¹;**
**X⁴** represents -H, **A¹-A²-,** or **A²-;**
**X⁵** represents -H, **-A⁹, -A⁹-A¹⁰,** or **-A⁹-A¹⁰-A¹¹;**
**X⁶** represents -H, or **A¹-;**
**X⁷** represents -H, **-A¹⁰,** or **-A¹⁰-A¹¹;**
**X⁸** represents -H, or **-A¹¹;**
**wherein**
**A¹** represents an amino acid selected from G and A;
**A²** represents an amino acid selected from Y, F, and W;
**A³** represents an amino acid selected from R, K and L;
**A⁴** represents an amino acid selected from G, and A;
**A⁵** represents an amino acid selected from I, Y, Q, N, L, and F;
**A⁶** represents an amino acid selected from S and T;
**A⁷** represents an amino acid selected from L, I, and V;
**A⁸** represents an amino acid selected from A, G, and V;
**A⁹** represents an amino acid selected from K, N, and Q;
**A¹⁰** represents an amino acid selected from W, and F;
**A¹¹** represents an amino acid selected from M, M-NH₂, L, I, V, A, and G;
and more preferably wherein
**A¹** represents an amino acid selected from G and A;
A² represents an amino acid selected from Y, and F;
A³ represents an amino acid selected from R, K and L;
A⁴ represents an amino acid selected from G, and A;
A⁵ represents an amino acid selected from I, and Y;
A⁶ represents an amino acid selected from S and T;
A⁷ represents an amino acid selected from L, and I;
A⁸ represents an amino acid selected from A, and G;
**A⁹** represents an amino acid selected from K and N;
**A¹⁰** represents an amino acid selected from W, and F;
**A¹¹** represents an amino acid selected from M, M-NH₂, L, I, and V;
and still more preferably wherein
**A'** represents G;
**A²** represents an amino acid selected from Y, and F;
**A³** represents an amino acid selected from R, K and L;
**A⁴** represents G;
**A⁵** represents an amino acid selected from I, and Y;
**A⁶** represents an amino acid selected from S and T;
**A⁷** represents L;
**A⁸** represents A;
**A⁹** represents an amino acid selected from K and N;
**A¹⁰** represents W;
**A¹¹** represents an amino acid selected from M, M-NH₂, and V.

Moreover, in preferred self-assembling peptides the residue
**A¹** represents G, while the residues **A² - A¹¹** have the meanings as disclosed herein, or
**A²** represents Y or F and most preferably Y, while the residues **A¹, A³ - A¹¹** have the meanings as disclosed herein,
   or
**A³** represents R or L and most preferably R, while the residues **A¹, A², A⁴ - A¹¹** have the meanings as disclosed herein,
   or
**A⁴** represents G, while the residues **A¹ - A**³ and **A⁵ - A¹¹** have the meanings as disclosed herein,
   or
**A⁵** represents I or Y and most preferably I, while the residues **A¹ - A⁴** and **A⁶** - **A¹¹** have the meanings as disclosed herein,
   or
**A⁶** represents S or T and most preferably S, while the residues **A¹** - **A⁵** and **A⁷ - A¹¹** have the meanings as disclosed herein,
   or
**A⁷** represents L, while the residues **A¹ - A**⁶ and **A⁸** - **A¹¹** have the meanings as disclosed herein,
   or
**A⁸** represents A, while the residues **A¹** - **A**⁷ and **A⁹** - **A¹¹** have the meanings as disclosed herein,
   or
**A⁹** represents N or K and most preferably N, while the residues **A' - A⁸, A¹⁰** and **A¹¹** have the meanings as disclosed herein,
   or
**A¹⁰** represents W, while the residues **A¹ - A⁹** and **A¹¹** have the meanings as disclosed herein,
   or
**A¹¹** represents M, M-NH₂, or V, more preferably M or V, still more preferably M or M-NH₂, and most preferably M, while the residues **A¹ - A¹⁰** have the meanings as disclosed herein.

Furthermore, the following self-assembling peptides consisting of at least 6 amino acids having the general formula (I) are preferred

**X¹-G-I-A⁶-X²**

**X¹-G-Y-A⁶-X²**

**X¹-G-A⁵-S-X²**

**X¹-G-A⁵-T-X²**

**X¹-A⁴-I-S-X²**

**X¹-A⁴-I-T-X²**

**X¹-A⁴-Y-S-X²**

**X¹-A⁴-Y-T-X²**

wherein
**X¹, X², A⁴, A⁵,** and **A⁶** have the meanings as discloses herein and preferably
**X¹** represents -H, **A¹-A²-A³- , A²-A³-** or **A³-;**
**X²** represents -H, **-A⁷, -A⁷-A⁸, -A⁷-A⁸-A⁹, -A⁷-A⁸-A⁹-A¹⁰,** or **-A⁷-A⁸-A⁹-A¹⁰-A¹¹;**
wherein
**A¹** represents an amino acid selected from G and A;
**A²** represents an amino acid selected from Y, F, and W;
**A³** represents an amino acid selected from R, K and L;
**A⁴** represents an amino acid selected from G, and A;
**A⁵** represents an amino acid selected from I, Y, Q, N, L, and F;
**A⁶** represents an amino acid selected from S and T;
**A⁷** represents an amino acid selected from L, I, and V;
**A⁸** represents an amino acid selected from A, G, and V;
**A⁹** represents an amino acid selected from K, N, and Q;
**A¹⁰** represents an amino acid selected from W, and F;
**A¹¹** represents an amino acid selected from M, M-NH₂, L, I, V, A, and G;
and more preferably wherein
**A'** represents an amino acid selected from G and A;
**A²** represents an amino acid selected from Y, and F;
**A³** represents an amino acid selected from R, K and L;
**A⁴** represents an amino acid selected from G, and A;
**A⁵** represents an amino acid selected from I, and Y;
**A⁶** represents an amino acid selected from S and T;
**A⁷** represents an amino acid selected from L, and I;
**A⁸** represents an amino acid selected from A, and G;
**A⁹** represents an amino acid selected from K and N;
**A¹⁰** represents an amino acid selected from W, and F;
**A¹¹** represents an amino acid selected from M, M-NH₂, L, I, and V;
and still more preferably wherein
**A¹** represents G;
**A²** represents an amino acid selected from Y, and F;
**A³** represents an amino acid selected from R, K and L;
**A⁴** represents G;
**A⁵** represents an amino acid selected from I, and Y;
**A⁶** represents an amino acid selected from S and T;
**A⁷** represents L;
**A⁸** represents A;
**A⁹** represents an amino acid selected from K and N;
**A¹⁰** represents W;
**A¹¹** represents an amino acid selected from M, M-NH₂, and V.

Furthermore, the following self-assembling peptides consisting of at least 6 amino acids having of general formula (I) are even more preferred

X¹-G-Y-S-X²

X¹-G-I-S-X²

X¹-G-Y-T-X²

**X¹-G-I-T-X²**

wherein
**X¹, X²** have the meanings as disclosed herein and preferably
**X¹** represents -H, **A¹-A²-A³- , A²-A³- or A³-;**
**X²** represents -H, **-A⁷, -A⁷-A⁸, -A⁷-A⁸-A⁹, -A⁷-A⁸-A⁹-A¹⁰,** or **-A⁷-A⁸-A⁹-A¹⁰-A¹¹;**
wherein
**A¹** represents an amino acid selected from G and A;
**A²** represents an amino acid selected from Y, F, and W;
**A³** represents an amino acid selected from R, K and L;
**A⁷** represents an amino acid selected from L, I, and V;
**A⁸** represents an amino acid selected from A, G, and V;
**A⁹** represents an amino acid selected from K, N, and Q;
**A¹⁰** represents an amino acid selected from W, and F;
**A¹¹** represents an amino acid selected from M, M-NH₂, L, I, V, A, and G;
and more preferably wherein
**A¹** represents an amino acid selected from G and A;
**A²** represents an amino acid selected from Y, and F;
**A³** represents an amino acid selected from R, K and L;
**A⁷** represents an amino acid selected from L, and I;
**A⁸** represents an amino acid selected from A, and G;
**A⁹** represents an amino acid selected from K and N;
**A¹⁰** represents an amino acid selected from W, and F;
**A¹¹** represents an amino acid selected from M, M-NH₂, L, I, and V;
and still more preferably wherein
**A¹** represents G;
**A²** represents an amino acid selected from Y, and F;
**A³** represents an amino acid selected from R, K and L;
**A⁷** represents L;
**A⁸** represents A;
**A⁹** represents an amino acid selected from K and N;
**A¹⁰** represents W;
**A¹¹** represents an amino acid selected from M, M-NH₂, and V.

Furthermore, self-assembling peptides consisting of at least 6 amino acids having the following general formulae are preferred:

**X¹-G-I-A⁶-L-X³**

**X¹-G-Y-A⁶-L-X³,**

**X¹-G-A⁵-S-L-X³,**

**X¹-G-A⁵-T-L-X³,**

**X¹-A ⁴-I-S-L-X³,**

**X¹-A⁴-1-T-L-X³,**

**X¹-A⁴-Y-S-L-X³,**

**X¹-A⁴-Y-T-L-X³**

**X¹-G-Y-S-L-X³** (SEQ ID NO: 31),
**X¹-G-I-S-L-X³** (SEQ ID NO: 32),
**X¹-G-Y-T-L-X³** (SEQ ID NO: 33),
**X¹-G-I-T-L-X³** (SEQ ID NO: 34),
**X¹-G-I-A⁶-L-A-X⁵** (SEQ ID NO: 35),
**X¹-G-Y-A⁶-L-A-X⁵** (SEQ ID NO: 36),
**X¹-G-A⁵-S-L-A-X⁵** (SEQ ID NO: 37),
**X¹-G-A⁵-T-L-A-X⁵** (SEQ ID NO: 38),
**X¹-A⁴-I-S-L-A-X⁵** (SEQ ID NO: 39),
**X¹-A⁴-I-T-L-A-X⁵** (SEQ ID NO: 40),
**X1-A⁴-Y-S-L-A-X⁵** (SEQ ID NO: 41),
**X¹-A⁴-Y-T-L-A-X⁵** (SEQ ID NO: 42),
**X¹-G-Y-S-L-A-X⁵** (SEQ ID NO: 43),
**X¹-G-I-S-L-A-X⁵** (SEQ ID NO: 44),
**X¹-G-Y-T-L-A-X⁵** (SEQ ID NO: 45),
**X¹-G-I- T -L-A-X⁵** (SEQ ID NO: 46),
**X¹-G-I-A⁶ -L-A-K-X⁷** (SEQ ID NO: 47),
**X¹-G-Y-A⁶-L-A-K-X⁷** (SEQ ID NO: 48),
**X¹-G-A⁵-S-L-A-K-X⁷** (SEQ ID NO: 49),
**X¹-G-A⁵-T-L-A-K-X⁷** (SEQ ID NO: 50),
**X¹-A⁴-I-S-L-A-K-X⁷** (SEQ ID NO: 51),
**X¹-A⁴-I-T-L-A-K-X⁷** (SEQ ID NO: 52),
**X¹-A⁴-Y-S-L-A-K-X⁷** (SEQ ID NO: 53),
**X¹-A⁴-Y-T-L-A-K-X⁷** (SEQ ID NO: 54),
**X¹-G-Y-S-L-A-K-X⁷** (SEQ ID NO: 55),
**X¹-G-I-S-L-A-K-X⁷** (SEQ ID NO: 56),
**X¹-G-Y-T-L-A-K-X⁷** (SEQ ID NO: 57),
**X¹-G-I-T-L-A-K-X⁷** (SEQ ID NO: 58),
**X¹-G-I-A⁶ -L-A-N-X⁷** (SEQ ID NO: 59),
**X¹-G-Y-A⁶-L-A-N-X⁷** (SEQ ID NO: 60),
**X¹-G-A⁵-S-L-A-N-X⁷** (SEQ ID NO: 61),
**X¹-G-A⁵-T-L-A-N-X⁷** (SEQ ID NO: 62),
**X¹-A⁴-I-S-L-A-N-X⁷** (SEQ ID NO: 63),
**X¹-A⁴-I-T-L-A-N-X⁷** (SEQ ID NO: 64),
**X¹-A⁴-Y-S-L-A-N-X⁷** (SEQ ID NO: 65),
**X¹-A⁴-Y-T-L-A-N-X⁷** (SEQ ID NO: 66),
**X¹-G-Y-S-L-A-N-X⁷** (SEQ ID NO: 67),
**X¹-G-I-S-L-A-N-X⁷** (SEQ ID NO: 68),
**X¹-G-Y-T-L-A-N-X⁷** (SEQ ID NO: 69),
**X¹-G-I-T-L-A-N-X⁷** (SEQ ID NO: 70),
**X¹-G-I-A⁶-L-A-N-W-X⁸** (SEQ ID NO: 71),
**X¹-G-Y-A⁶-L-A-N-W-X⁸** (SEQ ID NO: 72),
**X¹-G-A⁵-S-L-A-N-W-X⁸** (SEQ ID NO: 73),
**X¹-G-A⁵-T-L-A-N-W-X⁸** (SEQ ID NO: 74),
**X¹-A⁴-I-S-L-A-N-W-X⁸** (SEQ ID NO: 75),
**X¹-A⁴-I-T-L-A-N-W-X⁸** (SEQ ID NO: 76),
**X¹-A⁴-Y-S-L-A-N-W-X⁸** (SEQ ID NO: 77),
**X¹-A⁴-Y-T-L-A-N-W-X⁸** (SEQ ID NO: 78),
**X¹-G-Y-S-L-A-N-W-X⁸** (SEQ ID NO: 79),
**X¹-G-I-S-L-A-N-W-X⁸** (SEQ ID NO: 80),
**X¹-G-Y-T-L-A-N-W-X⁸** (SEQ ID NO: 81),
**X¹-G-I-T-L-A-N-W-X⁸** (SEQ ID NO: 82),
**X¹-G-I_A⁶-L-A-N-W-M** (SEQ ID NO: 83),
**X¹-G-Y-A⁶-L-A-N-W-M** (SEQ ID NO: 84),
**X¹-G-A⁵-S-L-A-N-W-M** (SEQ ID NO: 85),
**X¹-G-A⁵-T-L-A-N-W-M** (SEQ ID NO: 86),
**X¹-A⁴-I-S-L-A-N-W-M** (SEQ ID NO: 87),
**X¹-A⁴-I-T-L-A-N-W-M** (SEQ ID NO: 88),
**X¹-A⁴-Y-S-L-A-N-W-M** (SEQ ID NO: 89),
**X¹-A⁴-Y-T-L-A-N-W-M** (SEQ ID NO: 90),
**X¹-G-Y-S-L-A-N-W-M** (SEQ ID NO: 91),
**X¹-G-I-S-L-A-N-W-M** (SEQ ID NO: 92),
**X¹-G-Y-T-L-A-N-W-M** (SEQ ID NO: 93),
**X¹-G-I-T-L-A-N-W-M** (SEQ ID NO: 94),
**X¹-G-I-A⁶-L-A-N-W-M-NH₂** (SEQ ID NO: 95),
**X¹-G-Y-A⁶-L-A-N-W-M-NH₂** (SEQ ID NO: 96),
**X¹-G-A⁵-S-L-A-N-W-M-NH₂** (SEQ ID NO: 97),
**X¹-G-A⁵-T-L-A-N-W-M-NH₂** (SEQ ID NO: 98),
**X¹-A⁴-I-S-L-A-N-W-M-NH₂** (SEQ ID NO: 99),
**X¹-A⁴-I-T-L-A-N-W-M-NH₂** (SEQ ID NO: 100),
**X¹-A⁴-Y-S-L-A-N-W-M-NH₂** (SEQ ID NO: 111),
**X¹-A⁴-Y-T-L-A-N-W-M-NH₂** (SEQ ID NO: 112),
**X¹-G-Y-S-L-A-N-W-M-NH₂** (SEQ ID NO: 113),
**X¹-G-I-S-L-A-N-W-M-NH₂** (SEQ ID NO: 114),
**X¹-G-Y-T-L-A-N-W-M-NH2** (SEQ ID NO: 115),
**X¹-G-I-T-L-A-N-W-M-NH2** (SEQ ID NO: 116),
**X¹-G-I-A⁶-L-A-N-W-V** (SEQ ID NO: 117),
**X¹-G-Y-A⁶-L-A-N-W-V** (SEQ ID NO: 118),
**X¹-G-A⁵-S-L-A-N-W-V** (SEQ ID NO: 119),
**X¹-G-A⁵-T-L-A-N-W-V** (SEQ ID NO: 120),
**X¹-A⁴-I-S-L-A-N-W-V** (SEQ ID NO: 121),
**X¹-A⁴-I-T-L-A-N-W-V** (SEQ ID NO: 122),
**X¹-A⁴-Y-S-L-A-N-W-V** (SEQ ID NO: 123),
**X¹-A⁴-Y-T-L-A-N-W-V** (SEQ ID NO: 124),
**X¹-G-Y-S-L-A-N-W-V** (SEQ ID NO: 125),
**X¹-G-I-S-L-A-N-W-V** (SEQ ID NO: 126),
**X¹-G-Y-T-L-A-N-W-V** (SEQ ID NO: 127),
**X¹-G-I-T-L-A-N-W-V** (SEQ ID NO: 128),
**X⁴-R-G-I-A⁶-X²,**
**X⁴-R-G-Y-A⁶-X²,**
**X⁴-R-G-A⁵-S-X²,**
**X⁴-R-G-A⁵-T-X²,**
**X⁴-R-A⁴-I-S-X²,**
**X⁴-R-A⁴-I-T-X²,**
**X⁴-R-A⁴-Y-S-X²,**
**X⁴-R-A⁴-Y-T-X²,**
**X⁴-R-G-Y-S-X²** (SEQ ID NO: 129),
**X⁴-R-G-I-S-X²** (SEQ ID NO: 130),
**X⁴-R-G-Y-T-X²** (SEQ ID NO: 131),
**X⁴-R-G-I-T-X²** (SEQ ID NO: 132),

   **X⁴-K-G-I-A⁶-X²,**

   **X⁴-K-G-Y-A⁶-X²,**

   **X⁴-K-G-A⁵-S-X²,**

   **X⁴-K-G-A⁵-T-X²,**

   **X⁴-K-A⁴-I-S-X²**

   **X⁴-K-A⁴-1-T-X²,**

   **X⁴-K-A⁴-Y-S-X²,**

   **X⁴-K-A⁴-Y-T-X²,**
**X⁴-K-G-Y-S-X²** (SEQ ID NO: 133),
**X⁴-K-G-I-S-X²** (SEQ ID NO: 134),
**X⁴-K-G-Y-T-X²** (SEQ ID NO: 135),
**X⁴-K-G-I-T-X²** (SEQ ID NO: 136),

   **X⁴-L-G-I-A⁶-X²,**

   **X⁴-L-G-Y-A⁶-X²,**

   **X⁴-L-G-A⁵-S-X²,**

   **X⁴-L-G-A⁵-T-X²,**

   **X⁴-L-A⁴-I-S-X²,**

   **X⁴-L-A⁴-1-T-X²,**

   **X⁴-L-A⁴-Y-S-X²,**

   **X⁴-I-A⁴-Y-T-X²**
**X⁴-L-G-Y-S-X²** (SEQ ID NO: 137),
**X⁴-L-G-I-S-X²** (SEQ ID NO: 138),
**X⁴-L-G-Y-T-X²** (SEQ ID NO: 139),
**X⁴-L-G-I-T-X²** (SEQ ID NO: 140),
**X⁶-Y-R-G-I-A⁶-X²** (SEQ ID NO: 141),
**X⁶-Y-R-G-Y-A⁶-X²** (SEQ ID NO: 142),
**X⁶-Y-R-G-A⁵-S-X²** (SEQ ID NO: 143),
**X⁶-Y-R-G-A⁵-T-X²** (SEQ ID NO: 144),
**X⁶-Y-R-A⁴-I-S-X²** (SEQ ID NO: 145),
**X⁶-Y-R-A⁴-I-T-X²** (SEQ ID NO: 146),
**X⁶-Y-R-A⁴-Y-S-X²** (SEQ ID NO: 147),
**X⁶-Y-R-A⁴-Y-T-X²,** (SEQ ID NO: 148),
**X⁶-Y-R-G-Y-S-X²** (SEQ ID NO: 149),
**X⁶-Y-R-G-I-S-X²** (SEQ ID NO: 150),
**X⁶-Y-R-G-Y-T-X²** (SEQ ID NO: 151),
**X⁶-Y-R-G-I-T-X²** (SEQ ID NO: 152),
**X⁶-Y-K-G-I-A⁶-X²** (SEQ ID NO: 153),
**X⁶-Y-K-G-Y-A⁶-X²** (SEQ ID NO: 154),
**X⁶-Y-K-G-A⁵-S-X²** (SEQ ID NO: 155),
**X⁶-Y-K-G-A⁵-T-X²** (SEQ ID NO: 156),
**X⁶-Y-K-A⁴-I-S-X²** (SEQ ID NO: 157),
**X⁶-Y-K-A⁴-I-T-X²** (SEQ ID NO: 158),
**X⁶-Y-K-A⁴-Y-S-X²** (SEQ ID NO: 159),
**X⁶-Y-K-A⁴-Y-T-X²** (SEQ ID NO: 160),
**X⁶-Y-K-G-Y-S-X²** (SEQ ID NO: 161),
**X⁶-Y-K-G-I-S-X²** (SEQ ID NO: 162),
**X⁶-Y-K-G-Y-T-X²** (SEQ ID NO: 163),
**X⁶-Y-K-G-I-T-X²** (SEQ ID NO: 164),
**X⁶-Y-L-G-I-A⁶-X²** (SEQ ID NO: 165),
**X⁶-Y-L-G-Y-A⁶-X²** (SEQ ID NO: 166),
**X⁶-Y-L-G-A⁵-S-X²** (SEQ ID NO: 167),
**X⁶-Y-L-G-A⁵-T-X²** (SEQ ID NO: 168),
**X⁶-Y-L-A⁴-I-S-X²** (SEQ ID NO: 169),
**X⁶-Y-L-A⁴-I-T-X²** (SEQ ID NO: 170),
**X⁶-Y-L-A⁴-Y-S-X²** (SEQ ID NO: 171),
**X⁶-Y-L-A⁴-Y-T-X²** (SEQ ID NO: 172),
**X⁶-Y-L-G-Y-S-X²** (SEQ ID NO: 173),
**X⁶-Y-L-G-I-S-X²** (SEQ ID NO: 174),
**X⁶-Y-L-G-Y-T-X²** (SEQ ID NO: 175),
**X⁶-Y-L-G-I-T-X²** (SEQ ID NO: 176),
**X⁶-F-R-G-I-A⁶-X²** (SEQ ID NO: 177),
**X⁶-F-R-G-Y-A⁶-X²** (SEQ ID NO: 178),
**X⁶-F-R-G-A⁵-S-X²** (SEQ ID NO: 179),
**X⁶-F-R-G_A⁵-T-X²** (SEQ ID NO: 180),
**X⁶-F-R-A⁴-I-S-X²** (SEQ ID NO: 181),
**X⁶-F-R-A⁴-I-T-X²** (SEQ ID NO: 182),
**X⁶-F-R-A⁴-Y-S-X²** (SEQ ID NO: 183),
**X⁶-F-R-A⁴-Y-T-X²** (SEQ ID NO: 184),
**X⁶-F-R-G-Y-S-X²** (SEQ ID NO: 185),
**X⁶-F-R-G-I-S-X²** (SEQ ID NO: 186),
**X⁶-F-R-G-Y-T-X²** (SEQ ID NO: 187),
**X⁶-F-R-G-I-T-X²** (SEQ ID NO: 188),
**X⁶-F-K-G-I-A⁶-X²** (SEQ ID NO: 189),
**X⁶-F-K-G-Y-A⁶-X²** (SEQ ID NO: 190),
**X⁶-F-K-G-A⁵-S-X²** (SEQ ID NO: 191),
**X⁶-F-K-G-A⁵-T-X²** (SEQ ID NO: 192),
**X⁶-F-K-A⁴-I-S-X²** (SEQ ID NO: 193),
**X⁶-F-K-A⁴-I-T-X²** (SEQ ID NO: 194),
**X⁶-F-K-A⁴-Y-S-X²** (SEQ ID NO: 195),
**X⁶-F-K-A⁴-Y-T-X²** (SEQ ID NO: 196),
**X⁶-F-K-G-Y-S-X²** (SEQ ID NO: 197),
**X⁶-F-K-G-I-S-X²** (SEQ ID NO: 198),
**X⁶-F-K-G-Y-T-X²** (SEQ ID NO: 199),
**X⁶-F-K-G-I-T-X²** (SEQ ID NO: 200),
**X⁶-F-L-G-I-A⁶-X²** (SEQ ID NO: 310),
**X⁶-F-L-G-Y-A⁶-X²** (SEQ ID NO: 201),
**X⁶-F-L-G-A⁵-S-X²** (SEQ ID NO: 202),
**X⁶-F-L-G-A⁵-T-X²** (SEQ ID NO: 203),
**X⁶-F-L-A⁴-I-S-X²** (SEQ ID NO: 204),
**X⁶-F-L-A⁴-I-T-X²** (SEQ ID NO: 205),
**X⁶-F-L-A⁴-Y-S-X²** (SEQ ID NO: 206),
**X⁶-F-L-A⁴-Y-T-X²** (SEQ ID NO: 207),
**X⁶-F-L-G-Y-S-X²** (SEQ ID NO: 208),
**X⁶-F-L-G-I-S-X²** (SEQ ID NO: 209),
**X⁶-F-L-G-Y-T-X²** (SEQ ID NO: 210),
**X⁶-F-L-G-I-T-X²** (SEQ ID NO: 211),
**G-Y-R-G-I-A⁶-X²** (SEQ ID NO: 212),
**G-Y-R-G-Y-A⁶-X²** (SEQ ID NO: 213),
**G-Y-R-G-A⁵-S-X²** (SEQ ID NO: 214),
**G-Y-R-G-A⁵-T-X²** (SEQ ID NO: 215),
**G-Y-R-A⁴-I-S-X²** (SEQ ID NO: 216),
**G-Y-R-A⁴-I-T-X²** (SEQ ID NO: 217),
**G-Y-R-A⁴-Y-S-X²** (SEQ ID NO: 218),
**G-Y-R-A⁴-Y-T-X²** (SEQ ID NO: 219),
**G-Y-R-G-Y-S-X²** (SEQ ID NO: 220),
**G-Y-R-G-I-S-X²** (SEQ ID NO: 221),
**G-Y-R-G-Y-T-X²** (SEQ ID NO: 222),
**G-Y-R-G-I-T-X²** (SEQ ID NO: 223),
**G-Y-K-G-I-A⁶-X²** (SEQ ID NO: 224),
**G-Y-K-G-Y-A⁶-X²** (SEQ ID NO: 225),
**G-Y-K-G-A⁵-S-X²** (SEQ ID NO: 226),
**G-Y-K-G-A⁵-T-X²** (SEQ ID NO: 227),
**G-Y-K-A⁴-I-S-X²** (SEQ ID NO: 228),
**G-Y-K-A⁴-I-T-X²** (SEQ ID NO: 229),
**G-Y-K-A⁴-Y-S-X²** (SEQ ID NO: 230),
**G-Y-K-A⁴-Y-T-X²** (SEQ ID NO: 231),
**G-Y-K-G-Y-S-X²** (SEQ ID NO: 232),
**G-Y-K-G-I-S-X²** (SEQ ID NO: 233),
**G-Y-K-G-Y-T-X²** (SEQ ID NO: 234),
**G-Y-K-G-I-T-X²** (SEQ ID NO: 235),
**G-Y-L-G-I-A⁶-X²** (SEQ ID NO: 236),
**G-Y-L-G-Y-A⁶-X²** (SEQ ID NO: 237),
**G-Y-L-G-A⁵-S-X²** (SEQ ID NO: 238),
**G-Y-L-G-A⁵-T-X²** (SEQ ID NO: 239),
**G-Y-L-A⁴-I-S-X²** (SEQ ID NO: 240),
**G-Y-L-A⁴-I-T-X²** (SEQ ID NO: 241),
**G-Y-L-A⁴-Y-S-X²** (SEQ ID NO: 242),
**G-Y-L-A⁴-Y-T-X²** (SEQ ID NO: 243),
**G-Y-L-G-Y-S-X²** (SEQ ID NO: 244),
**G-Y-L-G-I-S-X²** (SEQ ID NO: 245),
**G-Y-L-G-Y-T-X²** (SEQ ID NO: 246),
**G-Y-L-G-I-T-X²** (SEQ ID NO: 247),
**G-F-R-G-I-A⁶-X²** (SEQ ID NO: 248),
**G-F-R-G-Y-A⁶-X²** (SEQ ID NO: 249),
**G-F-R-G-A⁵-S-X²** (SEQ ID NO: 250),
**G-F-R-G-A⁵-T-X²** (SEQ ID NO: 251),
**G-F-R-A⁴-I-S-X²** (SEQ ID NO: 252),
**G-F-R-A⁴-I-T-X²** (SEQ ID NO: 253),
**G-F-R-A⁴-Y-S-X²** (SEQ ID NO: 254),
**G-F-R-A⁴-Y-T-X²** (SEQ ID NO: 255),
**G-F-R-G-Y-S-X²** (SEQ ID NO: 256),
**G-F-R-G-I-S-X²** (SEQ ID NO: 257),
**G-F-R-G-Y-T-X²** (SEQ ID NO: 258),
**G-F-R-G-I-T-X²** (SEQ ID NO: 259),
**G-F-K-G-I-A⁶-X²** (SEQ ID NO: 260),
**G-F-K-G-Y-A⁶-X²** (SEQ ID NO: 261),
**G-F-K-G-A⁵-S-X²** (SEQ ID NO: 262),
**G-F-K-G-A⁵-T-X²** (SEQ ID NO: 263),
**G-F-K-A⁴-I-S-X²** (SEQ ID NO: 264),
**G-F-K-A⁴-I-T-X²** (SEQ ID NO: 265),
**G-F-K-A⁴-Y-S-X²** (SEQ ID NO: 266),
**G-F-K-A⁴-Y-T-X²** (SEQ ID NO: 267),
**G-F-K-G-Y-S-X²** (SEQ ID NO: 268),
**G-F-K-G-I-S-X²** (SEQ ID NO: 269),
**G-F-K-G-Y-T-X²** (SEQ ID NO: 270),
**G-F-K-G-I-T-X²** (SEQ ID NO: 271),
**G-F-L-G-I-A⁶-X²** (SEQ ID NO: 272),
**G-F-L-G-Y-A⁶-X²** (SEQ ID NO: 273),
**G-F-L-G-A⁵-S-X²** (SEQ ID NO: 274),
**G-F-L-G-A⁵-T-X²** (SEQ ID NO: 275),
**G-F-L-A⁴-I-S-X²** (SEQ ID NO: 276),
**G-F-L-A⁴-I-T-X²** (SEQ ID NO: 277),
**G-F-L-A⁴-Y-S-X²** (SEQ ID NO: 278),
**G-F-L-A⁴-Y-T-X²** (SEQ ID NO: 279),
**G-F-L-G-Y-S-X²** (SEQ ID NO: 280),
**G-F-L-G-I-S-X²** (SEQ ID NO: 281),
**G-F-L-G-Y-T-X²** (SEQ ID NO: 282),
**G-F-L-G-I-T-X²** (SEQ ID NO: 283),
**X⁴-R-G-I-A⁶-L-X³** (SEQ ID NO: 284),
**X⁴-R-G-Y-A⁶-L-X³** (SEQ ID NO: 285),
**X⁴-R-G-A⁵-S-L-X³** (SEQ ID NO: 286),
**X⁴-R-G-A⁵-T-L-X³** (SEQ ID NO: 287),
**X⁴-R-A⁴-I-S-L-X³** (SEQ ID NO: 288),
**X⁴-R-A⁴-I-T-L-X³** (SEQ ID NO: 289),
**X⁴-R-A⁴-Y-S-L-X³** (SEQ ID NO: 290),
**X⁴-R-A⁴-Y-T-L-X³** (SEQ ID NO: 291),
**X⁴-R-G-Y-S-L-X³** (SEQ ID NO: 292),
**X⁴-R-G-I-S-L-X³** (SEQ ID NO: 293),
**X⁴-R-G-Y-T-L-X³** (SEQ ID NO: 294),
**X⁴-R-G-I-T-L-X³** (SEQ ID NO: 295),
**X⁴-K-G-I-A⁶-L-X³** (SEQ ID NO: 296),
**X⁴-K-G-Y-A⁶-L-X³** (SEQ ID NO: 297),
**X⁴-K-G-A⁵-S-L-X³** (SEQ ID NO: 298),
**X⁴-K-G-A⁵-T-L-X³** (SEQ ID NO: 299),
**X⁴-K-A⁴-I-S-L-X³** (SEQ ID NO: 300),
**X⁴-K-A⁴-I-T-L-X³** (SEQ ID NO: 301),
**X⁴-K-A⁴-Y-S-L-X³** (SEQ ID NO: 302),
**X⁴-K-A⁴-Y-T-L-X³** (SEQ ID NO: 303),
**X⁴-K-G-Y-S-L-X³** (SEQ ID NO: 304),
**X⁴-K-G-I-S-L-X³** (SEQ ID NO: 305),
**X⁴-K-G-Y-T-L-X³** (SEQ ID NO: 306),
**X⁴-K-G-I-T-L-X³** (SEQ ID NO: 307),
**X⁴-L-G-I-A⁶-L-X³** (SEQ ID NO: 308),
**X⁴-L-G-Y-A⁶-L-X³** (SEQ ID NO: 309),
**X⁴-L-G-A⁵-S-L-X³** (SEQ ID NO: 101),
**X⁴-L-G-A⁵-T-L-X³** (SEQ ID NO: 102),
**X⁴-L-A⁴-I-S-L-X³** (SEQ ID NO: 103),
**X⁴-L-A⁴-I-T-L-X³** (SEQ ID NO: 104),
**X⁴-L-A⁴-Y-S-L-X³** (SEQ ID NO: 105),
**X⁴-L-A⁴-Y-T-L-X³** (SEQ ID NO: 106),
**X⁴-L-G-Y-S-L-X³** (SEQ ID NO: 107),
**X⁴-L-G-I-S-L-X³** (SEQ ID NO: 108),
**X⁴-L-G-Y-T-L-X³** (SEQ ID NO: 109),
**X⁴-L-G-I-T-L-X³** (SEQ ID NO: 110),
wherein
**X¹** represents -H, **A¹-A²-A³- , A²-A³- or A³-;**
**X²** represents -H, **-A⁷, -A⁷-A⁸, -A⁷-A⁸-A⁹, -A⁷-A⁸-A⁹-A¹⁰,** or **-A⁷-A⁸-A⁹**-**A¹⁰-A¹¹.**
**X³** represents -H, **-A⁸, -A⁸-A⁹, -A⁸-A⁹-A¹⁰,** or **-A⁸-A⁹-A¹⁰-A¹¹; X⁴** represents -H, **A¹-A²-,** or **A²-;**
**X⁵** represents -H, **-A⁹, -A⁹-A¹⁰,** or **-A⁹-A¹⁰-A¹¹;**
**X⁶** represents -H, or **A¹-;**
**X⁷** represents -H, **-A¹⁰,** or **-A¹⁰-A¹¹;**
**X⁸** represents -H, or **-A¹¹.**
wherein
**A¹** represents an amino acid selected from G and A;
**A²** represents an amino acid selected from Y, F, and W;
**A³** represents an amino acid selected from R, K and L;
**A⁴** represents an amino acid selected from G, and A;
**A⁵** represents an amino acid selected from I, Y, Q, N, L, and F;
**A⁶** represents an amino acid selected from S and T;
**A⁷** represents an amino acid selected from L, I, and V;
**A⁸** represents an amino acid selected from A, G, and V;
**A⁹** represents an amino acid selected from K, N, and Q;
**A¹⁰** represents an amino acid selected from W, and F;
**A¹¹** represents an amino acid selected from M, M-NH₂, L, I, V, A, and G;
and more preferably wherein
**A¹** represents an amino acid selected from G and A;
**A²** represents an amino acid selected from Y, and F;
**A³** represents an amino acid selected from R, K and L;
**A⁴** represents an amino acid selected from G, and A;
**A⁵** represents an amino acid selected from I, and Y;
**A⁶** represents an amino acid selected from S and T;
**A⁷** represents an amino acid selected from L, and I;
**A⁸** represents an amino acid selected from A, and G;
**A⁹** represents an amino acid selected from K and N;
**A¹⁰** represents an amino acid selected from W, and F;
**A¹¹** represents an amino acid selected from M, M-NH₂, L, I, and V;
and still more preferably wherein
**A¹** represents G;
**A²** represents an amino acid selected from Y, and F;
**A³** represents an amino acid selected from R, K and L;
**A⁴** represents G;
**A⁵** represents an amino acid selected from I, and Y;
**A⁶** represents an amino acid selected from S and T;
**A⁷** represents L;
**A⁸** represents A;
**A⁹** represents an amino acid selected from K and N;
**A¹⁰** represents W;
**A¹¹** represents an amino acid selected from M, M-NH₂, and V.

Preferred are self-assembling peptide consisting of the following amino acid sequence selected from: GYLGIS (SEQ ID NO: 2), GISLAN (SEQ ID NO: 3), YRGISLANWM (SEQ ID NO: 4), YRGISLANW (SEQ ID NO: 5), GFRGISLANWM (SEQ ID NO: 6), GFRGISLANWV (SEQ ID NO: 312), GFRGISLANWM-NH₂ (SEQ ID NO: 313), GYKGISLANWM (SEQ ID NO: 7), GYKGISLANWV (SEQ ID NO: 314), GYKGISLANWM-NH₂ (SEQ ID NO: 315), GYRGYSLANWM (SEQ ID NO: 9), GYRGYSLANWV (SEQ ID NO: 316), GYRGYSLANWM-NH₂ (SEQ ID NO: 317), GYRGITLANWΛΛ (SEQ ID NO: 10), GYRGITLANWV (SEQ ID NO: 318), GYRGITLANWM-NH₂ (SEQ ID NO: 319), GYRGISLAKWM (SEQ ID NO: 11) GYRGISLAKWV (SEQ ID NO: 320) GYRGISLAKWM-NH₂ (SEQ ID NO: 321) GYRGISLANWM (SEQ ID NO: 8), GYRGISLANWV (SEQ ID NO: 12), GYLGISLANW (SEQ ID NO: 13), and GYRGISLANWM-NH₂ (SEQ ID NO: 14).

Very preferred are the following sequences:
GYL-GIS (SEQ ID NO: 2),
GIS-LAN (SEQ ID NO: 3),
YR-GIS-LANWM (SEQ ID NO: 4),
YR-GIS-LANW (SEQ ID NO: 5),
GFR-GIS-LANWM (SEQ ID NO: 6),
GYK-GIS-LANWM (SEQ ID NO: 7),
GYR-GIS-LANWM (SEQ ID NO: 8),
GYR-GYS-LANWM (SEQ ID NO: 9),
GYR-GIT-LANWM (SEQ ID NO: 10),
GYR-GIS-LAKWM (SEQ ID NO: 11)
GYR-GIS-LANWV (SEQ ID NO: 12),
GYL-GIS-LANW (SEQ ID NO: 13), and
GYR-GIS-LANWM-NH₂ (SEQ ID NO: 14).

Especially preferred is sequence GYRGISLANWM (SEQ ID NO: 8).

The present application is also directed to a hydrogel composition comprising an aqueous dispersion phase comprising an aqueous dispersion medium; and at least one self-assembling peptide as disclosed herein, wherein the hydrogel is formed by non-covalent interactions of said self-assembling peptide in said aqueous dispersion phase.

Preferably the hydrogel compositions disclosed herein are hydrogel compositions, wherein the self-assembling peptide is present in said hydrogel at a level of from about 0.01% by weight to about 10% by weight, based upon the total weight of the hydrogel.

Moreover it is preferred that wherein the aqueous dispersion medium is physiologically acceptable.

This aqueous dispersion medium preferably comprises one or more salts selected from (NH₄)₂SO₄, Na₂SO₄, NaCl, KCl, CH₃COONH₄, NH₃C(CH₂OH)₃Cl, KH₂PO₄, and Na₂HPO₄.

The hydrogels disclosed herein have preferably a pH value between 4.0 and 8.5, more preferably between 4.5 and 8.0 or between 4.5 and 7.5 or between 4.5 and 7.0 or between 5.0 and 7.5 or between 5.0 and 7.0.

Moreover, the hydrogels disclosed herein have preferably an ionic strength between 0.0001 M and 1.5 M, preferably between 0.001 M and 0.5 M, more preferably 0.005 M and 0.3 M.

Furthermore, the hydrogels disclosed herein have preferably a storage modulus in the range of about 10 Pa to about 100,000 Pa.

The amino acids in the peptide chains may have any stereochemical orientation. For example, each amino acid may independently from each other be an L-amino acid or a D-amino acid. In one embodiment, all amino acids in the peptide chain are L-amino acids. In one embodiment, all amino acids in the peptide chain are D-amino acids.

The amino acids in the peptide chain may optionally be in their salt form. A person skilled in the art knows that this may depend, for example, on the environment of the peptide chain. For example, an acidic environment may lead to protonation of basic functional groups of the side chains of the amino acids. For example, a basic environment may lead to deprotonation of acidic functional groups of the side chains of the amino acids.

In preferred embodiments, the peptide chains form aggregates with each other.

In preferred embodiments, the peptide chains are self-assembling. Without wishing to be bound by theory: The peptide chains may form non-covalent bonds with each other. The peptide chains may form a supramolecular assembly. This may lead to non-covalent or supramolecular crosslinking, which may lead to gelation. In one embodiment, the peptide chains may form nanofibers or nanofibrillar structures. In one embodiment, the peptide chains may form beta sheets.

The peptide chains may optionally contain further amino acids. In further embodiments, at most 50%, preferably at most 40%, more preferably at most 30%, even more preferably at most 20%, even more preferably at most 10%, even more preferably at most 5%, particularly preferably at most 2%, of the amino acids in the peptide chain are further amino acids. In other embodiments, the peptide chains do not contain any further amino acids. Preferably, the further amino acids are amino acids other than proteinogenic amino acids. Preferably, the further amino acids are selected from fluorine-containing amino acids, hydroxy group-containing amino acids other than proteinogenic amino acids, boronic acid-containing amino acids, anthracenyl-containing amino acids, amino acids having C₂-C₁₀ alkyl groups other than proteinogenic amino acids, amino acids having C₂-C₁₀ alkenyl groups, and amino acids having C₂-C₁₀ alkynyl groups. Preferably, the further amino acids F are selected from azidohomoalanine, acridinylalanine, phenylselenocysteine, sulfoserine, p-iodophenylalanine, bipyridylalanine, dansylalanine, o-nitrobenzyl cysteine, 7-nitroindolinyl-amino acids, propargylglycine, azidonorleucine, 5-bromotryptophan, L-4'-deoxy-4'-iodophenylalanine, tetrazine alanine, dipyridyl tetrazine serine, hydroxyproline, beta-alanine, citrulline, ornithine, norleucine, 3-nitrotyrosine, nitroarginine, and pyroglutamic acid.

### Description of the Figures

**Figure 1****:** shows TEM images of the inventive self-assembling peptide with the sequence GYRGISLANWM (SEQ ID NO 8) at pH 4.75 (100 mM acetate buffer). **A:** 10 mg/mL. **B:** 1 mg/mL. TEM grid of 10 mg/mL sample was most densly covered amongst all samples
**Figure 2****:** shows TEM images of the inventive self-assembling peptide with the sequence GYRGISLANWM (SEQ ID NO 8) at pH 7.4 (DPBS). **A:** 10 mg/mL. **B:** 1 mg/mL.
**Figure 3****:** shows TEM images of the inventive self-assembling peptide with the sequence GYRGISLANWM (SEQ ID NO 8) at pH 8 (100 mM phosphate buffer). **A:** 10 mg/mL. **B:** 1 mg/mL. TEM images of GYRGISLANWM peptide at pH 8 (100mM phosphate buffer). Left: 10 mg/mL. Right: 1 mg/mL. At 1 mg/mL, no fibrils were observed at all.

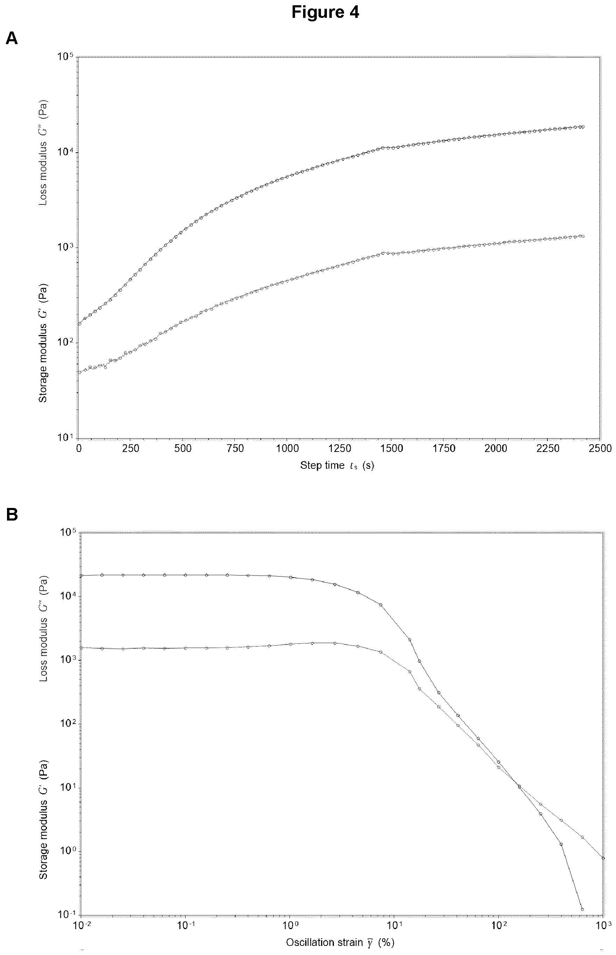

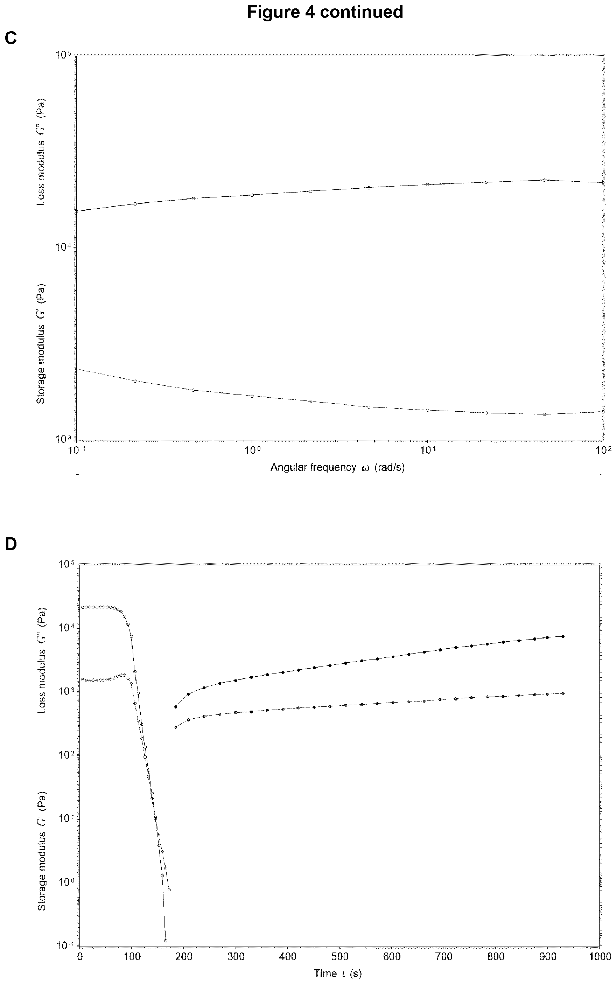

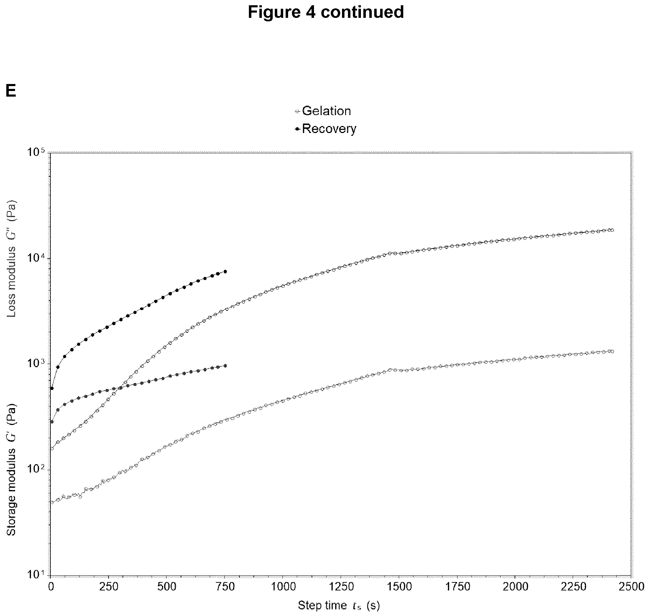

**Figure 4****:** Rheological characterization of hydrogels comprising the inventive self-assembling peptide with the sequence GYRGISLANWM (SEQ ID NO 8) at 10 mg/mL (1 wt%) in 100 mM acetate buffer, pH 4.75. Analyses include **A** time sweep measurement for monitoring of gelation kinetics, **B** strain sweep for monitoring gel-to-sol transition and shear thinning behavior, **C** frequency sweep to examine potential frequency dependent mechanical properties, as well as **D** consecutive time and strain sweep to analyze thixotropic behavior and gel recovery, with a comparative demonstration of gel formation during initial sample gelation and during recovery after gel-to-sol transition in the strain sweep **E.**
   **Figure 4B****:** Crossover-Strain for gel-to-sol transition is found to be 141%.
   **Figure 4D****:** Gel recovery after about 800s is around 34%.

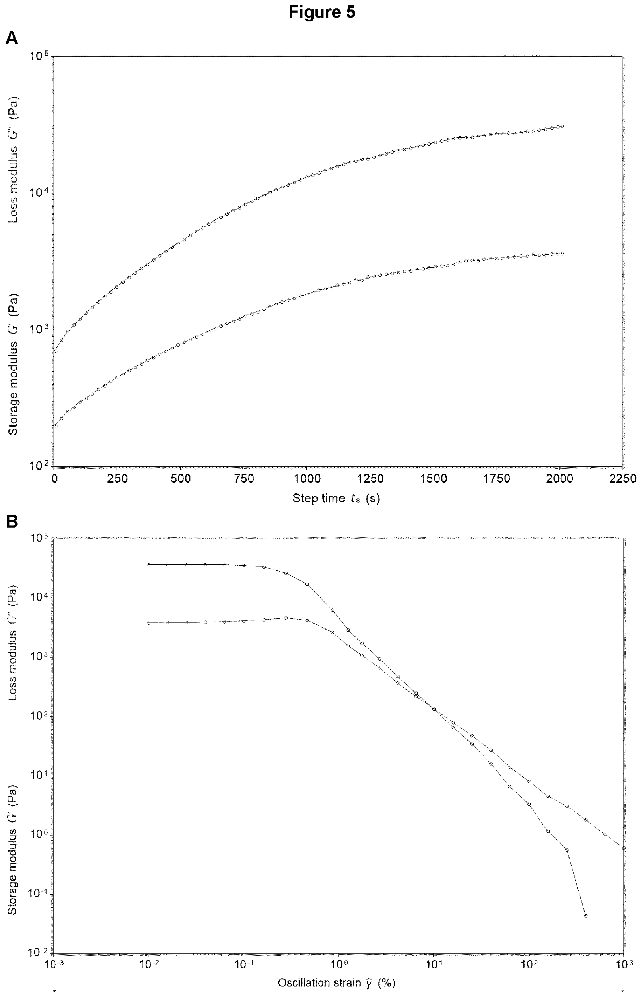

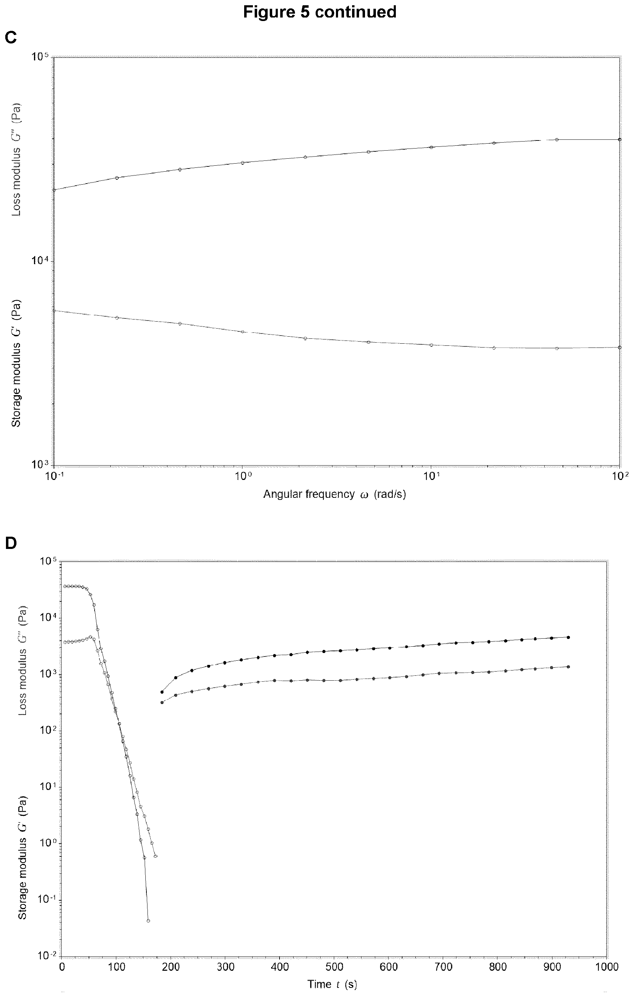

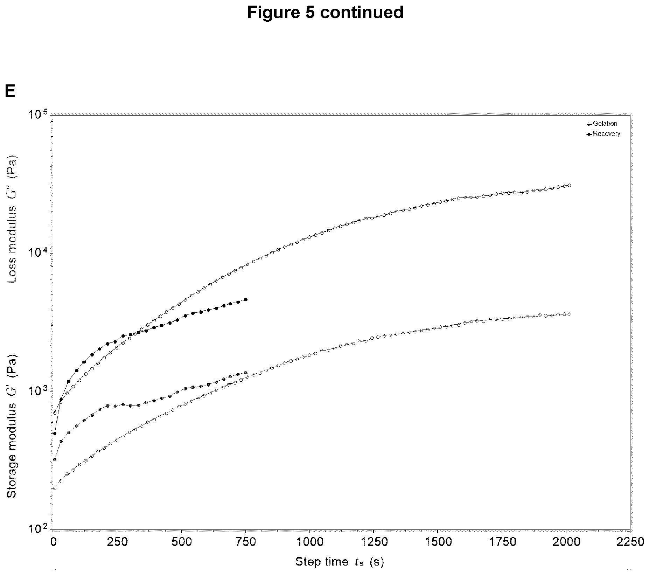

**Figure 5****:** Rheological characterization of hydrogels comprising the inventive self-assembling peptide with the sequence GYRGISLANWM (SEQ ID NO 8) at 10 mg/mL (1 wt%) in DPBS, pH 7.4. Analyses include **A** time sweep measurement for monitoring of gelation kinetics, **B** strain sweep for monitoring gel-to-sol transition and shear thinning behavior, **C** frequency sweep to examine potential frequency dependent mechanical properties, as well as **D** consecutive time and strain sweep to analyze thixotropic behavior and gel recovery, with a comparative demonstration of gel formation during initial sample gelation and during recovery after gel-to-sol transition in the strain sweep **E.**
   **Figure 5B****:** Crossover-Strain for gel-to-sol transition is found to be 9.6%.
   **Figure 5D****:** Gel recovery after about 800s is around 8%.

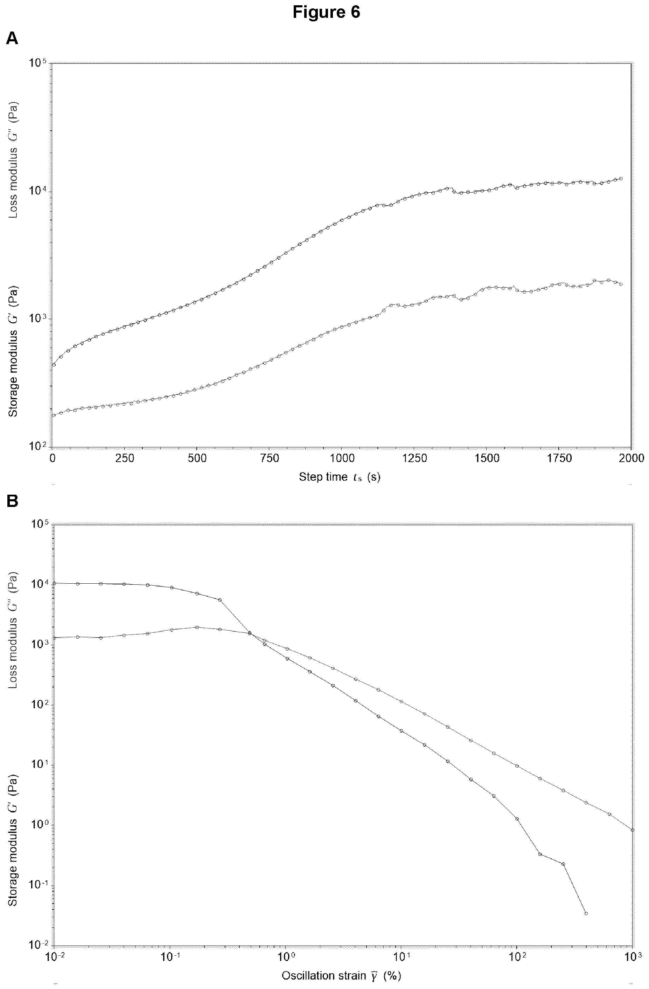

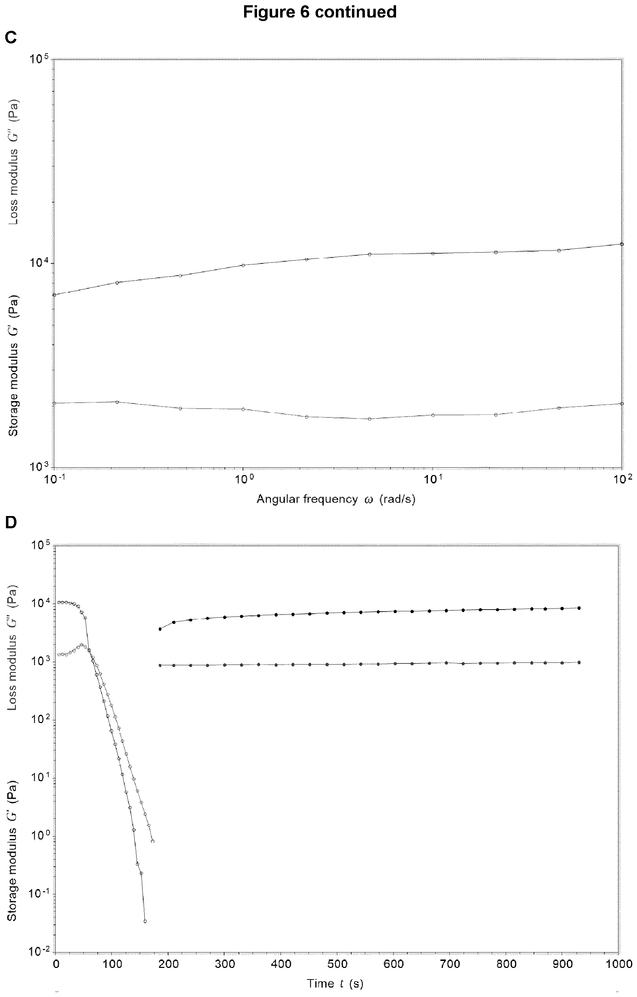

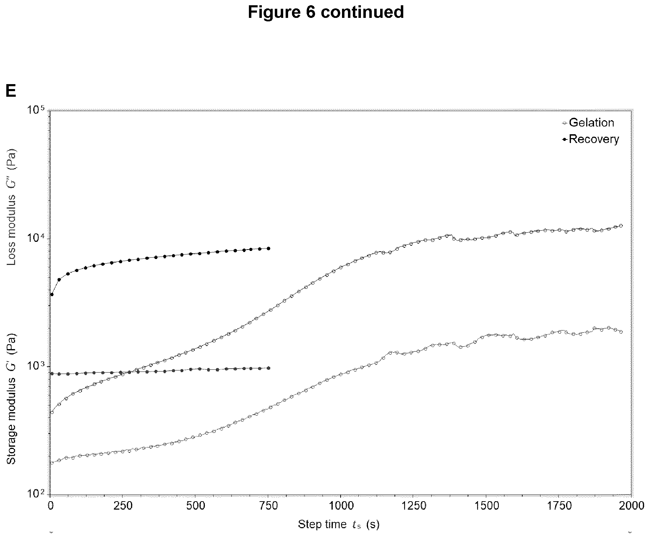

**Figure 6****:** Rheological characterization of hydrogels comprising the inventive self-assembling peptide with the sequence GYRGISLANWM (SEQ ID NO 8) at 10 mg/mL (1 wt%) in phosphate buffer 100mM, pH 8. Analyses include **A** time sweep measurement for monitoring of gelation kinetics, **B** strain sweep for monitoring gel-to-sol transition and shear thinning behavior, **C** frequency sweep to examine potential frequency dependent mechanical properties, as well as **D** consecutive time and strain sweep to analyze thixotropic behavior and gel recovery, with a comparative demonstration of gel formation during initial sample gelation and during recovery after gel-to-sol transition in the strain sweep **E.**
   **Figure 6B****:** Crossover-Strain for gel-to-sol transition is found to be 0.5%.
   **Figure 6D****:** Gel recovery after about 800s is around 70%.

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the spirit and scope of the invention.

Further modifications and alternative embodiments of various aspects of the invention will be apparent to those skilled in the art in view of this description. Accordingly, this description is to be construed as illustrative only and is for the purpose of teaching those skilled in the art the general manner of carrying out the invention. It is to be understood that the forms of the invention shown and described herein are to be taken as examples of embodiments. Elements and materials may be substituted for those illustrated and described herein, parts and processes may be reversed, and certain features of the invention may be utilized independently, all as would be apparent to one skilled in the art after having the benefit of this description of the invention. Changes may be made in the elements described herein without departing from the spirit and scope of the invention as described in the following claims.

### EXAMPLES

### Abbreviations and Acronyms

DPBS - Dulbecco's Phosphate Buffered Saline

### Methods

### Synthesis

The peptides were synthesized according to methods known in the art. All used amino acids are L-amino acids.

If not stated otherwise, RT refers to room temperature (20 - 25°C).

### Sample preparation:

Dissolving solid powder directly in respective solvent:
The peptide solid was dissolved directly in the respective solvent to yield a 1 mg/ml or 10 mg/mL solution/suspension or hydrogel. For example, 1 mg peptide solid was dissolved in 1 mL buffer (e.g., DPBS, pH 7.4) and mixed via vortex for approx. 5s to yield a 1 mg/mL solution/suspension. The sample was then incubated on a shaker at 500 rpm (22°C) for 24 h. To yield a 10 mg/mL hydrogel, 10 mg peptide solid were dissolved in 1 mL buffer (e.g., DPBS, pH 7.4) and mixed via vortex or pipetting for approx. 3s and incubated until gelation was completed or until further use with immediate start of gelation upon mixing with buffer.

### Transmission electron microscopy (TEM):

TEM was performed with aliquots of preincubated peptide samples. In order to assess the sample structures at 10 mg/mL, a dilution from 10 mg/mL was performed prior to imaging. The peptide was dissolved at 10 mg/mL in sterile filtered (0.22 µm filter unit) i) 100 mM acetate buffer pH 4.75, ii) DPBS pH 7.4, or iii) 100mM phosphate buffer pH 8 and after 1 hour incubation at room temperature diluted to 1 mg/mL in the respective solvent i-iii. For example, 0.17 mg of peptide were dissolved in 17 µL of the solvent i) to yield a 10 mg/mL solution/hydrogel. From this stock solution, 2 µL were transferred to 20 µL of solvent i) to yield a 1 mg/mL solution. 10 µL of the sample (a) 10 mg/mL as well as b) 1 mg/mL) were deposited on a copper grid coated with a thin electron-transparent Formvar-layer. Subsequent to an incubation time of 5 min, the sample solution was removed using filter paper, approx. 5 µL of an aqueous 4% uranyl acetate solution were added on a surface and the copper grid layed on top and left for incubation for 2.5 minutes to enhance sample contrast via staining. The grids were washed three times in Milli-Q-water, excess water was removed via filter paper and the grids were dried in air. Imaging was performed in high vacuum with a JEOL 1400 transmission electron microscope at an acceleration voltage of 120 kV equipped with a CCD camera.

### Rheology:

Rheological characterization was conducted using a DHR3 rheometer (TA Instruments) equipped with a temperature controller and a solvent reservoir to prevent hydrogel drying. Experiments were performed using an 8 mm parallel-plate geometry with hydrogels of approximately 30 µL volume resulting in a gap size of approximately 0.5 mm. Characterization of the hydrogel mechanical properties was conducted at 25 °C. Gels were prepared directly on the plate by adding buffer to the lyophilized peptide to achieve 10mg/mL using DPBS pH 7.4, 100 mM phosphate buffer pH 8, or 100mM acetate buffer pH 4.75.

Different experiments were performed:
**(i)** Time sweep: oscillatory time-sweep measurements were performed to monitor the gelation curves at a fixed strain of 0.1% and a fixed frequency of 1 Hz at 25°C until gelation was complete.
**(ii)** Strain sweep: Oscillatory strain sweeps (0.01-1000%) were conducted at a fixed frequency of 1 Hz at 25°C.
**(iii)** Frequency sweeps: Frequency sweeps (0.01-100 rad/s) were performed at a fixed strain of 0.1% at 25°C.
**(iv)** Thixotropy measurement: Consecutive measurements of an oscillatory strain sweep (0.01-1000%) at 25°C with a fixed frequency of 1 Hz followed by an oscillatory time sweep measurement with a fixed strain of 0.1% and frequency of 1 Hz for 800 s at 25°C were conducted to analyze the self-healing capacity of the hydrogels. In this context, the hydrogel recovery at the end of the consecutive oscillatory time sweep (0.1% strain, 800s) was analyzed comparing G' after the amplitude sweep with G' prior to it.

### Example 1

The self-assembling peptide of amino acid sequence GYRGISLANWM (SEQ ID NO: 8) was prepared and analyzed in accordance with the above described methods.

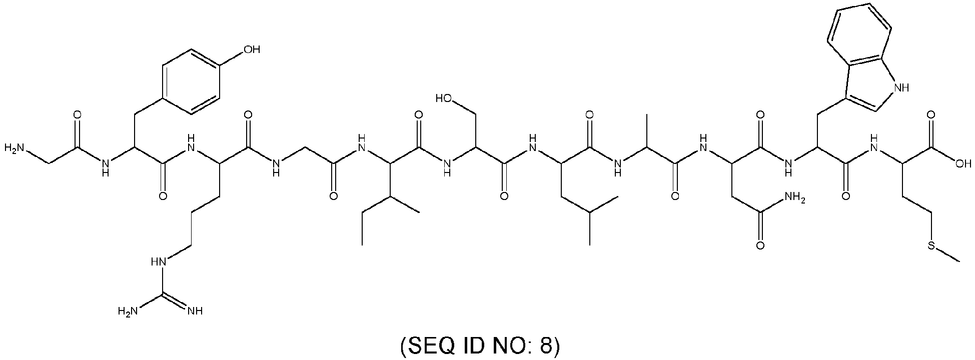

### Results:

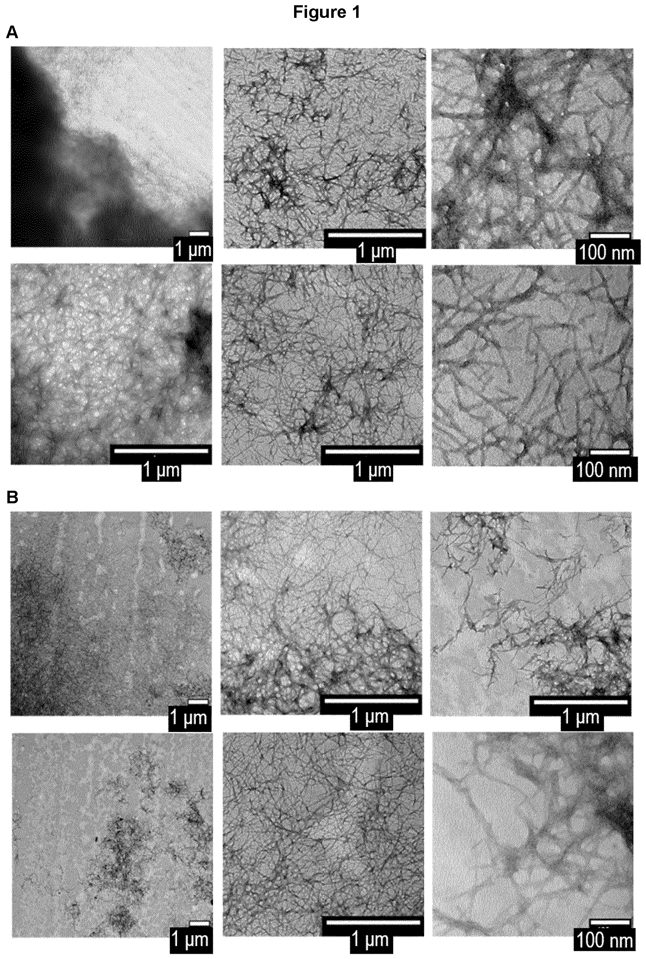

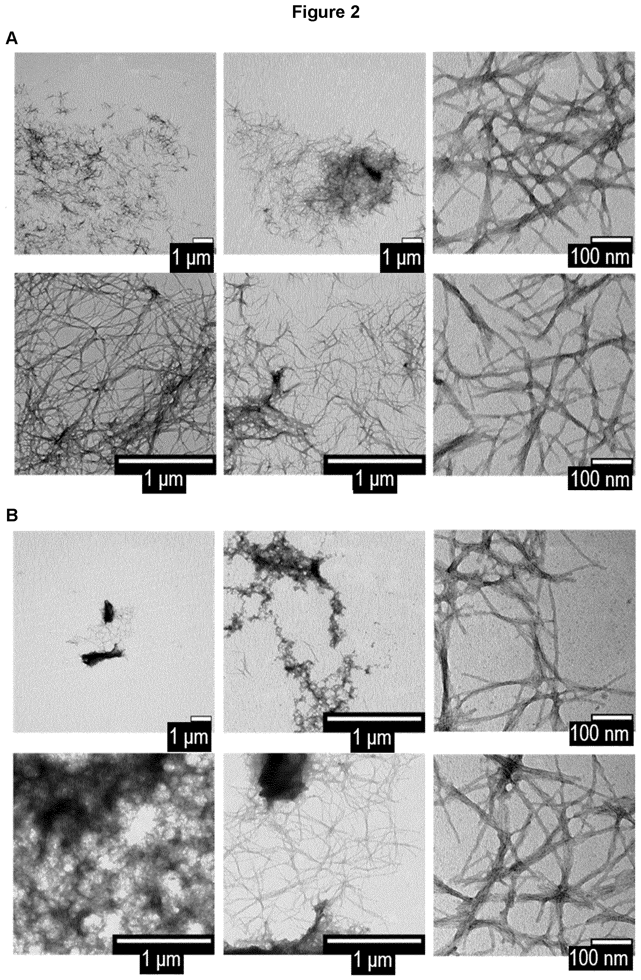

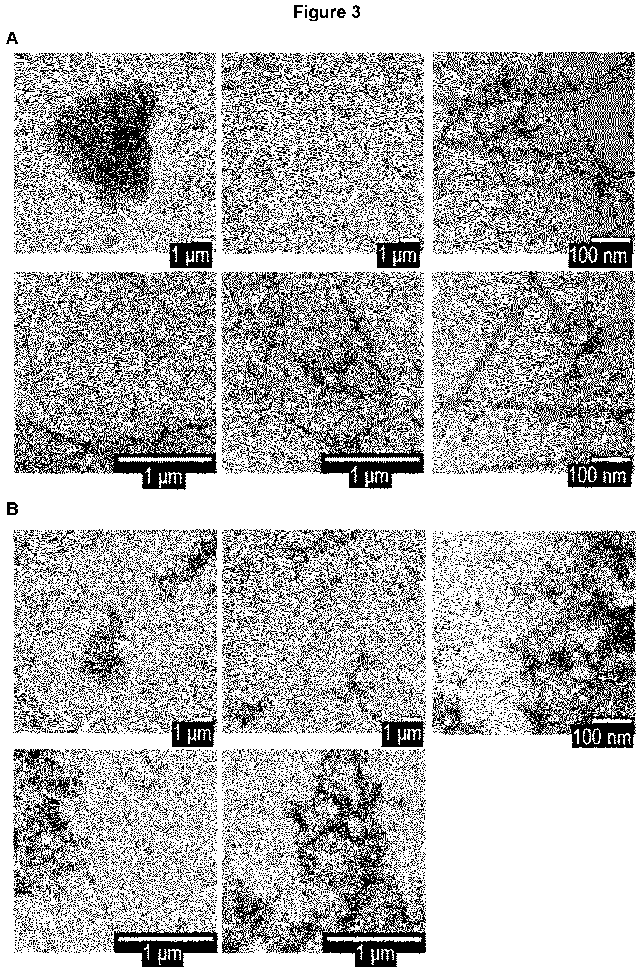

The TEM results are demonstrated in Figures 1 -3. Figure 1 shows TEM images of the inventive self-assembling peptide with the sequence GYRGISLANWM (SEQ ID NO 8) at pH 4.75 (100 mM acetate buffer). **A:** 10 mg/mL. **B:** 1 mg/mL. TEM grid of 10 mg/mL sample was most densly covered amongst all samples. Figure 2 shows TEM images of the inventive self-assembling peptide with the sequence GYRGISLANWM (SEQ ID NO 8) at pH 7.4 (DPBS). **A:** 10 mg/mL. **B:** 1 mg/mL. Figure 3 shows TEM images of the inventive self-assembling peptide with the sequence GYRGISLANWM (SEQ ID NO 8) at pH 8 (100 mM phosphate buffer). **A:** 10 mg/mL. **B:** 1 mg/mL. TEM grid of 10 mg/mL At 1 mg/mL, no fibrils were observed at all.

The results to the rheological characterization of hydrogels are demonstrated in Figures 4 -6. Figure 4 shows rheological characterization of hydrogels comprising the inventive self-assembling peptide with the sequence GYRGISLANWM (SEQ ID NO 8) at 10 mg/mL (1 wt%) in 100 mM acetate buffer, pH 4.75. Analyses include **A** time sweep measurement for monitoring of gelation kinetics, **B** strain sweep for monitoring gel-to-sol transition and shear thinning behavior, **C** frequency sweep to examine potential frequency dependent mechanical properties, as well as **D** consecutive time and strain sweep to analyze thixotropic behavior and **E** gel recovery after shear stress. Figure 5 shows Rheological characterization of hydrogels comprising the inventive self-assembling peptide with the sequence GYRGISLANWM (SEQ ID NO 8) at 10 mg/mL (1 wt%) in DPBS, pH 7.4. Analyses include **A** time sweep measurement for monitoring of gelation kinetics, **B** strain sweep for monitoring gel-to-sol transition and shear thinning behavior, **C** frequency sweep to examine potential frequency dependent mechanical properties, as well as **D** consecutive time and strain sweep to analyze thixotropic behavior and **E** gel recovery after shear stress. Figure 6 shows rheological characterization of hydrogels comprising the inventive self-assembling peptide with the sequence GYRGISLANWM (SEQ ID NO 8) at 10 mg/mL (1 wt%) in 100mM phosphate buffer, pH 8. Analyses include **A** time sweep measurement for monitoring of gelation kinetics, **B** strain sweep for monitoring gel-to-sol transition and shear thinning behavior, **C** frequency sweep to examine potential frequency dependent mechanical properties, as well as **D** consecutive time and strain sweep to analyze thixotropic behavior and **E** gel recovery after shear stress.

### Example 2

In addition to the self-assembling peptide of amino acid sequence GYRGISLANWM (SEQ ID NO: 8) of Example 1, further peptides have been analyzed (in accordance with the Methods as described above). The peptides are shown in table 1.

### Gelation behavior

**Table 1 Peptide screening for gelation in DPBS at 1 wt%.**

| SEQ ID NO | Sequence | Gelation | 10 Pa < G' < 100 Pa | 100 Pa < G' < 1000 Pa | 1000 G' > 10.000 Pa | 10.000 G' > 100.000 Pa |
|---|---|---|---|---|---|---|
| 4 | YRGISLANWM | yes | // | // | 3754 | // |
| 5 | YRGISLANW | yes | // | // | 1339 | // |
| 15 | YRGIS | G' < G" | // | // | // | // |
| 16 | SLANWM | G' = G" / G' < G" | // | // | // | // |
| 3 | GlSLAN | yes | // | // | // | 10.501 |
| 17 | ISLA | G' < G" | // | // | // | // |
| 18 | ISLAN | G' < G" | // | // | // | // |
| 19 | GYRGI | G' < G" | // | // | // | // |
| 20 | LANW | G' < G" | // | // | // | // |
| 21 | RGISL | G' < G" | // | // | // | // |
| 6 | GFRGISLANWM | yes | // | // | // | 11.135 |
| 22 | GIRGISLANIM | n/a | n/a | n/a | n/a | n/a |
| 23 | GKRGISLANWM | G' < G" | // | // | // | // |
| 7 | GYKGISLANWM | yes | // | // | 2163 | // |
| 24 | GYVGISLANWM | n/a | n/a | n/a | n/a | n/a |
| 25 | GYEGISLANWM | n/a | n/a | n/a | n/a | n/a |
| 9 | GYRGYSLANWM | yes | // | 361 | // | // |
| 10 | GYRGITLANWM | yes | // | // | // | 40.926 |
| 26 | GYRGISFANWM | n/a | n/a | n/a | n/a | n/a |
| 11 | GYRGISLAKWM | yes | 73 | // | // | // |
| 27 | GYRGISLAEWM | n/a | n/a | n/a | n/a | n/a |
| 12 | GYRGISLANWV | yes | // | 281 | // | // |
| 28 | KYKGISLANWM | G' = G" / G' < G" | // | // | // | // |
| 29 | GYRGISLFNWM | n/a | n/a | n/a | n/a | n/a |
| 2 | GYLGIS | yes | // | // | 1092 | // |
| 13 | GYLGISLANW | yes | // | // | 1788 | // |
| 30 | GISLAL | n/a | n/a | n/a | n/a | n/a |
| 14 | GYRGISLANWM* | yes | // | 911 | // | // |

| | | | | | | |
|---|---|---|---|---|---|---|
| *C - Terminal: Amidation n/a: insoluble | | | | | | |

## Claims

1. A self-assembling peptide consisting of at least 6 amino acids having the following amino acid sequence of general formula (I):
**X¹-A⁴-A⁵-A⁶-X²** **(I)**
wherein
**X¹** represents -H, **A³-, A²-A³- or A¹-A²-A³- ;**
**X²** represents -H, **-A⁷, -A⁷-A⁸, -A⁷-A⁸-A⁹, -A⁷-A⁸-A⁹-A¹⁰,** or **-A⁷-A⁸-A⁹-A¹⁰-A¹¹;**
**A¹** and **A⁴** represent independently of each other a small neutral amino acid or a neutral hydrophilic amino acid;
**A²** and **A⁵** represent independently of each other a neutral amino acid, an aromatic amino acid or an amino acid amide;
**A³** represents a basic amino acid, a large neutral amino acid, or an amino acid amide;
**A⁶** represents an amino acid selected from S and T;
**A⁷** and **A⁸** represent independently of each other a neutral amino acid;
**A⁹** represents a basic amino acid, or an amino acid amide;
**A¹⁰** represents a basic amino acid, or an aromatic amino acid;
**A¹¹** represents a neutral amino acid, a hydrophilic amino acid, or an amino acid amide.

2. The self-assembling peptide according to claim 1, wherein
**A¹** represents an amino acid selected from G and A;
**A²** represents an amino acid selected from Y, F, and W;
**A³** represents an amino acid selected from R, K and L;
**A⁴** represents an amino acid selected from G, and A;
**A⁵** represents an amino acid selected from I, Y, Q, N, L, and F;
**A⁶** represents an amino acid selected from S and T;
**A⁷** represents an amino acid selected from L, I, and V;
**A⁸** represents an amino acid selected from A, G, and V;
**A⁹** represents an amino acid selected from K, N, and Q;
**A¹⁰** represents an amino acid selected from W, and F;
**A¹¹** represents an amino acid selected from M, M-NH₂, L, I, V, A, and G.

3. The self-assembling peptide according to claim 1, wherein
**A¹** represents an amino acid selected from G and A;
**A²** represents an amino acid selected from Y, and F;
**A³** represents an amino acid selected from R, K and L;
**A⁴** represents an amino acid selected from G, and A;
**A⁵** represents an amino acid selected from I, and Y;
**A⁶** represents an amino acid selected from S and T;
**A⁷** represents an amino acid selected from L, and I;
**A⁸** represents an amino acid selected from A, and G;
**A⁹** represents an amino acid selected from K and N;
**A¹⁰** represents an amino acid selected from W, and F;
**A¹¹** represents an amino acid selected from M, M-NH₂, L, I, and V.

4. The self-assembling peptide according to claim 1, wherein
**A¹** represents G;
**A²** represents an amino acid selected from Y, and F;
**A³** represents an amino acid selected from R, K and L;
**A⁴** represents G;
**A⁵** represents an amino acid selected from I, and Y;
**A⁶** represents an amino acid selected from S and T;
**A⁷** represents L;
**A⁸** represents A;
**A⁹** represents an amino acid selected from K and N;
**A¹⁰** represents W;
**A¹¹** represents an amino acid selected from M, M-NH₂, and V.

5. The self-assembling peptide according to any one of claim 1 to 4, wherein
**X¹** represents **A¹-A²-A³- or A²-A³-;** and
**X²** represents **-A⁷-A⁸-A⁹, -A⁷-A⁸-A⁹-A¹⁰,** or **-A⁷-A⁸-A⁹-A¹⁰-A¹¹.**

6. The self-assembling peptide according to claim 1 consisting of the following amino acid sequence selected from: GYLGIS (SEQ ID NO: 2), GISLAN (SEQ ID NO: 3), YRGISLANWM (SEQ ID NO: 4), YRGISLANW (SEQ ID NO: 5), GFRGISLANWM (SEQ ID NO: 6), GFRGISLANWV (SEQ ID NO: 312), GFRGISLANWM-NH₂ (SEQ ID NO: 313), GYKGISLANWM (SEQ ID NO: 7), GYKGISLANWV (SEQ ID NO: 314), GYKGISLANWM-NH₂ (SEQ ID NO: 315), GYRGYSLANWM (SEQ ID NO: 9), GYRGYSLANVW (SEQ ID NO: 316), GYRGYSLANWM-NH₂ (SEQ ID NO: 317), GYRGITLANWM (SEQ ID NO: 10), GYRGITLANWV (SEQ ID NO: 318), GYRGITLANWM-NH₂ (SEQ ID NO: 319), GYRGISLAKWM (SEQ ID NO: 11) GYRGISLAKWV (SEQ ID NO: 320) GYRGISLAKWM-NH₂ (SEQ ID NO: 321) GYRGISLANWM (SEQ ID NO: 8), GYRGISLANWV (SEQ ID NO: 12), GYLGISLANW (SEQ ID NO: 13), and GYRGISLANWM-NH₂ (SEQ ID NO: 14).

7. The self-assembling peptide according to claim 1 having the following amino acid sequence:
GYL-GIS (SEQ ID NO: 2),
GIS-LAN (SEQ ID NO: 3),
YR-GIS-LANWM (SEQ ID NO: 4),
YR-GIS-LANW (SEQ ID NO: 5),
GFR-GIS-LANWM (SEQ ID NO: 6),
GYK-GIS-LANWM (SEQ ID NO: 7),
GYR-GIS-LANWM (SEQ ID NO: 8),
GYR-GYS-LANWM (SEQ ID NO: 9),
GYR-GIT-LANWM (SEQ ID NO: 10),
GYR-GIS-LAKWM (SEQ ID NO: 11)
GYR-GIS-LANWV (SEQ ID NO: 12),
GYL-GIS-LANW (SEQ ID NO: 13), and
GYR-GIS-LANWM-NH₂ (SEQ ID NO: 14).

8. A hydrogel composition comprising an aqueous dispersion phase comprising an aqueous dispersion medium; and at least one self-assembling peptide according to any one of claims 1 to 7, wherein the hydrogel is formed by noncovalent interactions of said self-assembling peptide in said aqueous dispersion phase.

9. The hydrogel composition according to claim 8, wherein the self-assembling peptide is present in said hydrogel at a level of from about 0.01 % by weight to about 10% by weight, based upon the total weight of the hydrogel.

10. The hydrogel composition according to claim 8 or 9 wherein the aqueous dispersion medium is physiologically acceptable.

11. The hydrogel composition according to any one of claims 8 to 10, wherein the aqueous dispersion medium comprises one or more salts selected from (NH₄)₂SO₄, Na₂SO₄, NaCl, KCl, CH₃COONH₄, NH₃C(CH₂OH)₃Cl, KH₂PO₄, and Na₂HPO₄.

12. The hydrogel according to any one of claims 8 to 11 having a pH value between 4.0 and 8.5, preferably between 4.5 and 8.0.

13. The hydrogel according to any one of claims 8 to 12 having an ionic strength between 0.0001 M and about 1.5 M.

14. The hydrogel according to any one of claims 8 to 13 having a storage modulus in the range of about 10 Pa to about 100,000 Pa.
